# EUROPÄISCHE PATENTSCHRIFT

(11) **EP 2 736 461 B1**
(45) Veröffentlichungstag und Bekanntmachung des Hinweises auf die Patenterteilung: **30.08.2017**
(21) Anmeldenummer: 12713076.3
(22) Anmeldetag: 28.03.2012
(51) Int. Cl.: A61F 5/01, A43B 7/26

(54) **ORTHOPÄDISCHE VORRICHTUNG, INSBESONDERE ZUR KORREKTUR VON ZEHENFEHLSTELLUNGEN**
ORTHOPAEDIC DEVICE, IN PARTICULAR FOR CORRECTING DEFORMATIONS OF THE TOES
DISPOSITIF D'ORTHOPÉDIE, DESTINÉ NOTAMMENT À LA CORRECTION DE DÉFORMATIONS DES ORTEILS

(30) Priorität: 15.06.2011 DE 102011051083
(43) Veröffentlichungstag der Anmeldung: 04.06.2014
(73) Patentinhaber: Huber, Vitus Maria, 80638 München (DE)
(72) Erfinder: Huber, Vitus Maria, 80638 München (DE)
(74) Vertreter: Oberdorfer, Jürgen
(86) Internationale Anmeldenummer: PCT/EP2012/055560
(87) Internationale Veröffentlichungsnummer: WO 2012/171675

(56) Entgegenhaltungen:
- DE-A1- 2 916 894
- DE-U1- 9 206 317
- US-A- 353 910
- US-A- 1 245 468

## Beschreibung

Die Erfindung betrifft eine orthopädische Vorrichtung, insbesondere zur Korrektur von Zehenfehlstellungen gemäß dem Oberbegriff des Anspruchs 1.

Aus dem Stand der Technik sind bereits diverse orthopädische Vorrichtungen bekannt, die bei der Therapie einer Zehenfehlstellung zur Anwendung kommen. Dabei kann man zunächst zwischen Einlegesohlen oder Einlagen einerseits und Schienen andererseits unterscheiden. Bei Einlegesohlen/Einlagen ist die zu behandelnde Person darauf angewiesen, diese in einem Schuh zu tragen, da derartige Einlegesohlen/Einlagen, um funktionieren zu können, zwingend in einem Schuh eingelegt werden müssen. Eine zufriedenstellende Funktion derartiger Sohlen ist durch diese alleine ohne weitere Hilfsmittel, wie z. B. Schuhe, nicht erreichbar. Es ist oftmals nicht vorgesehen, die Einlage oder Einlegesohlen an dem zu behandelnden Fuß zu befestigen.

Ein Beispiel für eine derartige Sohle findet sich in der DE 198 45 884 A1. Die dort beschriebene Vorrichtung zum Korrigieren einer Fehlstellung einer Zehe umfasst eine Basis, die sich bei bestimmungsgemäßem Gebrauch im Wesentlichen unter der Fußsohle erstreckt. Des Weiteren sind Halteflansche an der Fußinnen- und der Fußaußenseite vorgesehen, so dass mittels eines Korrekturflansches eine Krafteinwirkung auf die große Zehe erfolgen kann, wodurch der Zehenfehlstellung entgegengewirkt wird. Die offenbarte Sohle ist offensichtlich für den Einsatz in einem Schuh vorgesehen, da eine Fixierung der Sohle am Fuß, die z. B. ein Gehen mit dieser Sohle ohne Schuh ermöglichen würde, nicht vorgesehen ist.

Neben derartigen Sohlen sind aus dem Stand der Technik des Weiteren Fußschienen bekannt, die beispielsweise mittels Bandagen an dem betroffenen Fuß fixiert werden können. Auch hier gibt es wiederum unterschiedliche Lösungsansätze bezüglich der Art und Weise, wie eine Krafteinwirkung auf die zu korrigierende Zehe ermöglicht werden soll.

Sowohl die DE 85 29 083 U1 als auch die DE 85 32 303 U1 offenbaren eine Schiene bzw. Spange, die jeweils eine Art Hebelarm aufweist, welcher in einem Gelenk gelagert ist. Das Gelenk befindet sich bei bestimmungsgemäßem Gebrauch unterhalb der Fußsohle in einem Bereich zwischen dem Mittelfuß und der Zehen. Die Schiene wird dabei zum einen im Bereich des Mittelfußes indirekt über einen Halter bzw. einen Halteausleger an dem Fuß befestigt. Zum anderen ist eine weitere Befestigung, z.B. eine Stützwand vorgesehen, mittels der über den Hebelarm eine Krafteinwirkung auf die zu korrigierende Zehe möglich ist. Derartige Schienen haben jedoch große Nachteile. Zum einen bestehen große Probleme mit dem Tragekomfort, da sich das Gelenk unterhalb der Fußsohle befindet, was von Anwendern als unangenehm empfunden wird. Zum anderen ist ein Gelenk grundsätzlich ein verschleißbehaftetes Bauteil, insbesondere wenn die Schiene beispielsweise im Freien getragen werden soll, wodurch Schmutz oder Sand in das Gelenk gelangen kann. Diese Nachteile gilt es zu vermeiden.

Zusätzlich wird es bei den in diesen Druckschriften offenbarten Schienen als nachteilig angesehen, dass die im Bereich des Mittelfußes wirkende Kraft und die auf die zu korrigierende Zehe wirkende Kraft voneinander abhängig sind. Es ist somit nicht möglich, die Kraft im Bereich des Mittelfußes zu erhöhen, beispielsweise wenn ein stärkeres Aufrichten des Mittelfußgewölbes nötig ist, ohne auch die auf die zu korrigierende Zehe wirkende Kraft zu verändern. Somit ist es mit diesen Schienen nicht möglich, zwei Symptome unabhängig voneinander zu behandeln, da immer ein "Kompromiss" aus den wirkenden Kräften geschlossen werden muss, was im Zweifelsfall dazu führt, dass keine der beiden Kräfte für die jeweilige Ausprägung des Symptoms optimal ist.

Eine Alternative zu derartigen Schienen wird beispielsweise in der DE 1 881 215 offenbart. Dort ist eine Ballenschiene beschrieben, die zweiteilig ausgebildet ist. Bei bestimmungsgemäßem Gebrauch werden die zwei Teile mittels Schlaufen aneinander befestigt, wobei der eine Teil an dem zu korrigierenden Zehen und der andere Teil an der Ferse befestigt ist. Da es insbesondere bei einer Hallux-Valgus-Fehlstellung zu einer sogenannten Pseudoexostose, also einer ballenförmigen Ausbuchtung im Bereich des Großzehengrundgelenks kommen kann, weist die offenbarte Schiene eine Wölbung in diesem Bereich auf, mittels der verhindert werden soll, dass die ballenförmige Ausbuchtung mit der Schiene in Berührung kommt. Diese Wölbung führt jedoch dazu, dass ein Tragen der vorgeschlagenen Schiene in einem Schuh nicht möglich, zumindest jedoch erschwert ist. Zusätzlich wird die Fixierung an der Ferse mittels einer Fersenspange als unangenehm empfunden.

Aus der US-Patentschrift 933,423 ist eine aus Federstahl hergestellte Korrekturvorrichtung bekannt, die im Schuh getragen wird. Die Korrekturvorrichtung besteht dabei im Wesentlichen aus einer an die Fußkontur angepassten Metallplatte, wobei zusätzlich ein Lederband vorgesehen ist, mit dem die Schiene an der zu korrigierenden Zehe befestigt wird. Um eine Korrekturkraft zur Fußinnenseite hin auf die zu korrigierende Zehe ausüben zu können, muss diese Vorrichtung in einem Schuh getragen werden, so dass sie im Sinne obiger Definition als Sohle anzusehen ist. Um eine natürliche Beugung der Zehen ansatzweise zu ermöglichen, ist die Metallplatte im Bodenbereich teilweise eingeschnitten. Dieser Einschnitt führt jedoch dazu, dass beim Laufen Haut in diesem Einschnitt eingeklemmt werden kann, was als außerordentlich nachteilig anzusehen ist. Des Weiteren lehrt diese Schrift zur Reduzierung des Schmerzempfindens im Bereich der Pseudoexostose diese abzuschirmen und zu panzern, damit dieser Bereich des Fußes nicht mit dem Schuhwerk in Kontakt kommt.

Eine Weiterentwicklung dieser Vorrichtung ist aus der US-Patentschrift 1,183,062 bekannt. Die dort offenbarte Schiene weist zum einen eine Auswölbung auf, durch die der Ballen und der Bereich des Zehengelenks aufgenommen und vor Druck geschützt wird. Dies führt jedoch wiederum dazu, dass ein Tragen dieser Schiene in einem Schuh schwierig oder sogar unmöglich wird. Zum anderen ist bei dieser Schiene anstelle des Einschnitts ein Scharnier vorgesehen, welches sich bei bestimmungsgemäßen Gebrauch unterhalb des Großzehengrundgelenks befindet. Wie auch die Gelenke der oben genannten Schienen ist auch das Scharnier anfällig für Schmutz oder Sand, was bei dieser Schiene als besonders nachteilig angesehen wird.

Aus der DE 102 40 121 A1 ist des Weiteren eine Gelenkbiegeschiene bekannt, welche in der Flexions-Extensionsrichtung des zu korrigierenden Zehs gelenkig ausgebildet ist. Hierzu weist die Schiene zwei Gelenkschienenschenkel auf, die durch ein Gelenk miteinander verbunden sind und von denen einer im Bereich des Mittelfußes an dem Fuß und der andere an der zu korrigierenden Zehe befestigt ist. Die Schiene erstreckt sich in ihrer Gesamtheit entlang der Fußinnenseite. Das Gelenk besitzt eine Gelenkachse, die möglichst genau mit der biologischen "Gelenkachse" der Großzehe fluchten soll. Zu diesem Zweck ist das Gelenk auf der Fußinnenseite angeordnet und befindet sich somit genau in dem Bereich, in dem üblicherweise die schmerzempfindliche Pseudoexostose liegt.

Aus der US 353,910 ist eine gattungsgemäße orthopädische Vorrichtung bekannt.

Es ist daher Aufgabe der Erfindung, eine wirkungsvolle Korrekturvorrichtung in Form einer Schiene bereitzustellen, die sowohl im Schuh, als auch ohne Schuh, sowohl nachts als auch tagsüber, sowohl im Freien als auch zu Hause, also im Wesentlichen unabhängig von den Umgebungsbedingungen getragen werden kann und durch die der Druck auf den Ballen und insbesondere auf den fußinnenseitigen Bereich, in dem sich üblicherweise die Pseudoexostose befindet, vermindert oder zumindest stark reduziert wird.

Eine weitere Aufgabe der Erfindung ist es, eine Schiene bereitzustellen, die keine komplizierte und/oder verschleißanfällige Mechanik aufweist.

Eine weitere Aufgabe einer Alternative der Erfindung ist es, die von mehreren, bisher bekannten Schienen verwendete Befestigungsschlaufe an der Zehe zu vermeiden, da diese oftmals im Zehengrund reibt und Blasen verursacht.

Eine weitere Aufgabe der Erfindung ist es, zur Aufrichtung des Mittelfußgewölbes beizutragen.

Diese Aufgaben werden mit einer orthopädischen Vorrichtung mit den Merkmalen des Anspruchs 1 gelöst. Vorteilhafte Weiterbildungen sind Gegenstand der Unteransprüche.

Die erfindungsgemäße orthopädische Vorrichtung wird insbesondere zur Korrektur von Zehenfehlstellungen eingesetzt und weist einen Halteschenkel, eine erste Befestigungseinrichtung, zumindest einen Korrekturschenkel sowie eine plattenartige Basisstruktur auf. Die plattenartige Basisstruktur verbindet dabei den Halteschenkel und den Korrekturschenkel. Mit der ersten Befestigungseinrichtung wird der Halteschenkel im Bereich des Mittelfußes an dem Fuß, an dem die Vorrichtung angebracht werden soll, befestigt. Der zumindest eine Korrekturschenkel dient zum unmittelbaren oder zum mittelbaren Einwirken auf die Großzehe.

Im Sinne der Erfindung ist im Folgenden als unmittelbares Einwirken auf die zu korrigierende Zehe, insbesondere die Großzehe, zu verstehen, wenn der Korrekturschenkel unmittelbar selbst durch drückendes Anliegen an der zu korrigierenden Zehe auf diese eine Korrekturkraft in Form einer Druckkraft aufbringt. Dabei muss zum unmittelbaren Einwirken des Korrekturschenkels auf die Großzehe dieser zwar nicht direkt auf der blanken Haut der Großzehe anliegen. Es können vielmehr Zwischenlagen zwischen dem Korrekturschenkel beispielsweise in Form von Stofflagen oder Polsterungen oder dergleichen vorhanden sein. Wesentlich für die unmittelbare Einwirkung des Korrekturschenkels auf den zu korrigierenden Zehen ist, dass dieser gegebenenfalls über Zwischenlagen eine Druckkraft auf den Großzeh ausübt. Demgegenüber benötigt das mittelbare Einwirken des Korrekturschenkels auf die Großzehe zur Übertragung der Korrekturkraft beispielsweise ein Zugmittel, nämlich eine Zehenbandage oder eine Zehenschlaufe, die die Korrekturkraft vom Korrekturschenkel der Schiene auf die Zehe überträgt. Ein derartiges mittelbares Einwirken des Korrekturschenkels und somit der gesamten Korrekturvorrichtung auf den Großzeh erfordert also ein weiteres kraftübertragendes Element, ohne dieses eine Kraftübertragung auf den zu korrigierenden Zeh nicht bewerkstelligt werden könnte.

Zur Lösung der eingangs genannten Aufgaben weist die erfindungsgemäße Vorrichtung einen Randkonturverlauf auf, der bei bestimmungsgemäßem Gebrauch einen fußinnenseitigen Seitenbereich des Großzehengrundgelenks, in dem üblicherweise eine schmerzempfindliche, insbesondere druckempfindliche Pseudoexostose angeordnet ist, frei lässt. Auf diese Weise wird Druck auf diesen Bereich im Vergleich zum Stand der Technik deutlich vermindert oder sogar ganz verhindert. Dies ist insbesondere bei einer Hallux-Valgus-Fehlstellung der Großzehe von Vorteil. Die Fußinnenseite ist dabei die Längsseite eines Fußes, welche zum benachbarten Fuß weist. Die Fußaußenseite ist die der Fußinnenseite gegenüberliegende Längsseite des Fußes.

Der Halteschenkel, der Korrekturschenkel und die Basisstruktur sind einstückig ausgebildet. Auf diese Weise wird zum einen die Stabilität der erfindungsgemäßen Vorrichtung deutlich erhöht und zum anderen können Verbindungselemente, insbesondere Scharniere oder Gelenke, vermieden werden. Die Vermeidung von derartigen Bauteilen führt, wie bereits beschrieben, zum einen dazu, dass sich der Tragekomfort maßgeblich erhöht, da das einstückig ausgebildete Bauteil an die Fußform bzw. die Fußkontur angepasst werden kann. Ein besonderer Vorteil der erfindungsgemäßen Vorrichtung besteht darin, dass sie im Allgemeinen relativ dünn ausgebildet sein kann und trotzdem die geforderte Stabilität aufweist. Im Gegensatz zu den bekannten Schienen aus dem Stand der Technik trägt die erfindungsgemäße Vorrichtung nicht stark auf und kann daher bequem in einem Schuh getragen werden.

Die ausgeprägte Stabilität der erfindungsgemäßen Vorrichtung ergibt sich insbesondere aus der plattenartigen Basisstruktur und deren einstückige Verbindung mit dem Halteschenkel und dem Korrekturschenkel. Dadurch ist die erfindungsgemäße Vorrichtung um eine im Wesentlichen senkrecht zu der natürlichen Auflagefläche des Fußes liegenden Achse sehr biegesteif, was insbesondere zur Stabilität der erfindungsgemäßen Vorrichtung beiträgt und das Erzeugen der erforderlichen Korrekturkraft - wie weiter unten beschrieben - ermöglicht. Die von der erfindungsgemäßen Vorrichtung auf dem Mittelfuß und die zu korrigierende Zehe aufgebrachten Kräfte können alleine durch die Biegesteifigkeit ausgeglichen werden. Somit ist es möglich, diese aufgebrachten Kräfte unabhängig voneinander an die Gegebenheiten anzupassen, da auch eine Veränderung einer der Kräfte von der erfindungsgemäßen Vorrichtung und deren Biegesteifigkeit aufgenommen werden kann, wodurch diese Veränderung keinen oder nur geringen Einfluss auf die jeweilige andere Kraft hat.

Weiterhin ist aufgrund der plattenartigen, insbesondere dünnwandigen Ausbildung der Basisstruktur eine Biegbarkeit der gesamten Korrekturvorrichtung in Flexions-Extensions-Richtung des Großzehs gewährleistet. In dieser Biege-/Beugerichtung, die im Wesentlichen der natürlichen Beugerichtung des Großzehs entspricht, ist die Korrekturvorrichtung somit biegeweich ausgebildet, so dass sie sich beim Abrollen des Fußes im Schuh somit an eine winklige Stellung der Zehe/Zehen relativ zum Mittelfuß ohne Weiteres anpassen kann. Im Ergebnis ist die Korrekturvorrichtung also wie oben beschrieben um eine Achse, die in etwa senkrecht auf der Aufstandsfläche des Fußes steht, besonders biegesteif ausgebildet, um eine Korrekturkraft zum Einwirken auf die Großzehe bereitzustellen. Um eine möglichst ungehinderte Bewegung des Großzehs in Flexions-Extensions-Richtung zu gewährleisten ist die Schiene durch die plattenartige, insbesondere auch elastische, biegbare Ausgestaltung der Basisstruktur um eine Achse in etwa parallel zur Aufstandsfläche des Fußes besonders biegeweich ausgebildet. Diese Anforderungen gelingen durch eine dünnwandige plattenartige Ausbildung der Basisstruktur, die somit auch in Flexions-Extensions-Richtung eine Biegezone bildet.

Zudem fördert die einstückige Ausführung die Langlebigkeit der erfindungsgemäßen Vorrichtung, da Verbindungselemente, wie beispielsweise Gelenke oder Scharniere, meist eine Schwachstelle darstellen und insbesondere empfindlich gegenüber Schmutz, Sand und dergleichen sind. Nicht zuletzt wird durch die einstückige Ausführung des Halteschenkels, der Basisstruktur und des Korrekturschenkels die Herstellung der erfindungsgemäßen Vorrichtung, insbesondere im Kunststoffspritzgussverfahren, vereinfacht. Zwar führt die einstückige Ausführung dazu, dass eine erfindungsgemäße Vorrichtung jeweils nur an dem linken oder dem rechten Fuß einsetzbar ist, dennoch ist die erfindungsgemäße Vorrichtung gegenüber bisher bekannten Schienen aufgrund der einstückigen Ausführung deutlich einfacher und kostengünstiger herzustellen.

Im Rahmen der Erfindung bezeichnet der Begriff "proximal" entsprechend der allgemeinen medizinischen Definition einen rumpfwärts in Richtung des Beins liegenden Bereich des Fußes. Der Begriff "distal" bedeutet demnach das genaue Gegenteil, also einen weiter vom Rumpf oder vom Bein entfernten Bereich.

Die Hallux-Valgus-Fehlstellung zeichnet sich insbesondere dadurch aus, dass die Großzehe aus ihrer natürlichen Position heraus in Richtung der restlichen Zehen des betroffenen Fußes weisend fehlgestellt ist. Dabei kommt es jedoch oftmals auch zu der bereits angesprochenen Pseudoexostose in Form einer ballenförmigen Ausbuchtung im fußinnenseitigen Bereich des Großzehengrundgelenks. Dadurch wird dieser Bereich des Fußes sehr schmerzempfindlich, insbesondere druckempfindlich. Daher lässt der Randkonturverlauf der erfindungsgemäßen Vorrichtung vorzugsweise mindestens den Bereich frei, d.h. unüberdeckt, in dem sich bei einer Hallux-Valgus-Fehlstellung eine derartige Pseudoexostose üblicherweise bilden kann, wodurch die erfindungsgemäße Vorrichtung eine deutliche Verbesserung hinsichtlich des Tragekomforts darstellt.

Bei einer weiteren alternativen Lösung der erfindungsgemäßen Aufgabe ist der Korrekturschenkel nicht einstückig mit der Basisstruktur verbunden, sondern als separates, mit der Basisstruktur lösbar verbindbares Korrekturelement ausgebildet. Der Kerngedanke der vorliegenden Erfindung, welcher durch den Randkonturverlauf der erfindungsgemäßen Vorrichtung, der bei bestimmungsgemäßem Gebrauch einen fußinnenseitigen Seitenbereich des Großzehengrundgelenks, in dem üblicherweise eine schmerzempfindliche, insbesondere druckempfindliche Pseudoexostose angeordnet ist, realisiert wird, ist auch bei dieser alternativen Lösung vorhanden. Bei dieser Lösung ist in der Basisstruktur zumindest eine Montageeinrichtung vorhanden.

Bei einer Ausführung der erfindungsgemäßen Vorrichtung ist die Montageeinrichtung als Montageausnehmung ausgebildet, wobei sie die Basisstruktur von der Oberseite zur Unterseite vollständig durchbricht. Sie kann aber auch auf der Oberseite als Vertiefung ausgebildet sein. Eine Hybridlösung, bei der die Montageeinrichtung im Wesentlichen als Vertiefung ausgebildet ist, jedoch in einem Teilbereich die Basisstruktur vollständig durchbricht, ist ebenfalls möglich.

Bei einer vorteilhaften Weiterbildung dieser erfindungsgemäßen Lösung ist eine Mehrzahl von Montageeinrichtungen vorgesehen, die alle so ausgebildet sind, dass das Korrekturelement in diesen fixierbar ist. Dies ermöglicht es, das Korrekturelement an verschiedenen Positionen der Basisstruktur anzuordnen, wodurch wiederum eine Anpassung der erfindungsgemäßen Vorrichtung - sowohl was die natürliche, unterschiedliche Ausbildung der Zehen von verschiedenen Menschen, als auch was die unterschiedliche Ausprägung der Hallux-Valgus-Fehlstellung betrifft - ermöglicht wird. Die Fixierung des Korrekturelements in der Montageeinrichtung kann beispielsweise mittels einer Presspassung oder einer lösbaren Clips-Lösung realisiert werden.

Gleichwohl ist es auch möglich, mit nur einer Montageeinrichtung eine variable Positionierung des Korrekturschenkels zu ermöglichen. Bei einer vorteilhaften Weiterbildung der erfindungsgemäßen Lösung sind dementsprechend mehrere unterschiedliche Korrekturelemente vorgesehen, die zwar alle die Montageeinrichtung vollständig ausfüllen, wobei sich die Anordnung des Korrekturschenkels auf dem Korrekturelement jedoch unterscheidet. Mit dieser Weiterbildung kann nun wahlweise das Korrekturelement ausgewählt werden, das zu der Fußform des zu behandelnden Fußes und der Ausprägung der Hallux-Valgus-Fehlstellung am besten passt, d.h. zu einem bestmöglichen Heilverlauf führt.

Bei einer vorteilhaften Weiterbildung der erfindungsgemäßen Vorrichtung ist die Montageeinrichtung als Vorsprung, insbesondere als Dorn, ausgebildet, auf dem das Korrekturelement, bevorzugt lösbar, befestigt ist. Hierzu ist in dem Korrekturelement eine mit dem Vorsprung korrespondierende Vertiefung vorgesehen. Hier eignet sich insbesondere eine Clips-Lösung, bei der das Korrekturelement Widerhaken aufweist, die sich mit der Montageeinrichtung verhaken und so eine ausreichend starke Fixierung ermöglichen. Die Widerhaken können so ausgebildet sein, dass bei Druck auf das Korrekturelement die Verhakung zumindest soweit gelöst wird, dass ein Entfernen des Korrekturelements möglich ist.

Die in dem Korrekturelement vorgesehene Vertiefung ist bei einer vorteilhaften Weiterbildung bei bestimmungsgemäßen Gebrauch nicht zentriert bezüglich des Korrekturelements vorgesehen. Vielmehr weist sie zu einem ersten Rand des Korrekturelements einen Abstand A und zu einem entgegen gesetzten zweiten Rand einen Abstand B auf, wobei gilt A > B. Die Vertiefung und die Montageeinrichtung sind dabei so ausgeführt, dass das Korrekturelement in zwei verschiedenen Lagen an der Basisstruktur befestigbar ist. Dabei ist in einer ersten Lage der erste Rand ausgehend von der Vertiefung in die Richtung, in die sich die Zehen erstrecken, ausgerichtet. In einer zweiten Lage ist der zweite Rand in diese Richtung ausgerichtet. Dies führt dazu, dass das Korrekturelement in der ersten Lage eine längere Erstreckung in der Richtung, in der sich die Zehen erstrecken, als in der zweiten Lage aufweist. Die Erstreckung entgegen dieser Richtung ist in der ersten Lage kleiner als in der zweiten Lage. Auf diese Weise kann die erfindungsgemäße Vorrichtung auf die Fußform des zu behandelnden Fußes angepasst werden. Insbesondere kann vermieden werden, dass das Korrekturelement den Zehengrund und insbesondere den Übergang zwischen der Großzehe und der benachbarten Zehe verletzt oder wund scheuert.

Bei einer besonders vorteilhaften Weiterbildung der erfindungsgemäßen Vorrichtung sind in der Basisstruktur Durchbrüche vorgesehen, die sowohl als schlitzförmige Öffnung, als auch als Montageeinrichtung dienen können. Dies ermöglicht es, dass die erfindungsgemäße Vorrichtung wahlweise entweder mittels einer durch die Durchbrüche geführten zweiten Befestigungseinrichtung oder mittels eines in einem Durchbruch angeordneten Korrekturelements auf die Großzehe einwirkt.

Bei einer vorteilhaften Weiterbildung der erfindungsgemäßen Vorrichtung, bei der schlitzförmige Öffnungen oder Montageeinrichtungen in der Basisstruktur vorgesehen sind, sind Einsteckelemente vorgesehen, die in unbenutzte Öffnungen oder Montageeinrichtungen einsteckbar sind. Sind beispielsweise fünf Öffnungen vorgesehen, so sind in der Regel drei unbenutzt, da für die Führung der zweiten Befestigungseinrichtung lediglich zwei erforderlich sind. Da sich die Öffnungen unterhalb der Zehen befinden, können unbenutzte Öffnungen nachteilig sein, da sich die Zehen an den Kanten der Öffnungen wund scheuern können. Die Einsteckelemente sind so ausgebildet, dass sie die Öffnungen oder Montageeinrichtungen vollständig ausfüllen, wodurch eine plane Auflagefläche für die Zehen gebildet wird. Besonders vorteilhaft ist es, wenn alle Öffnungen oder Montageeinrichtungen die gleiche Form aufweisen, sodass die Einsteckelemente identisch ausgestaltet werden können, wodurch die Herstellung einfacher und kostengünstiger wird. Alternativ ist es auch möglich, die Kanten der Öffnungen oder Montageeinrichtungen abzurunden oder aus einem weichen Material auszubilden um ein wund scheuern der Zehen zu vermeiden. Ebenso können die Einsteckelemente auf einem weichen Material ausgebildet sein, was den Tragekomfort erhöht.

Die Öffnungen und/oder Montageeinrichtungen sind bei einer vorteilhaften Weiterbildung der erfindungsgemäßen Vorrichtung nicht parallel zueinander angeordnet, sondern folgen vielmehr der Abknickbewegung der Großzehe, die diese durch die Hallux-Valgus-Fehlstellung erfährt. Vorteilhafterweise sind die Öffnungen und/oder Montageeinrichtungen also so angeordnet, dass zu verschiedenen Stellungen der Großzehe immer eine Öffnung und/oder Montageeinrichtung im Wesentlichen parallel zu der Großzehe angeordnet ist.

Um den Tragekomfort weiter zu verbessern und gleichzeitig für eine ausreichende Stabilität der erfindungsgemäßen Vorrichtung zu sorgen, erstreckt sich bei einer vorteilhaften Weiterbildung der Erfindung die Basisstruktur bei bestimmungsgemäßem Gebrauch in Querrichtung des Fußes gesehen zumindest unterhalb des Zehengrundgelenks der Großzehe. Es ist jedoch auch möglich und besonders vorteilhaft, dass sich die Basisstruktur bei bestimmungsgemäßem Gebrauch sowohl unterhalb des Zehengrundgelenks der Großzehe als auch des Zehengrundgelenks der Kleinzehe sowie einem dazwischenliegenden Bereich erstreckt. Somit erstreckt sich die erfindungsgemäße Vorrichtung bevorzugt über die gesamte Quererstreckung des Fußes unterhalb der Zehengrundgelenke. Dies führt zu einer weiteren Stabilitätsverbesserung der erfindungsgemäßen Vorrichtung und erhöht insbesondere die Biegesteifigkeit der Schiene um die Hochachse. Als Hochachse ist dabei eine Achse zu verstehen, die im Wesentlichen senkrecht zur Fußaufstandsfläche steht. Des Weiteren ist durch eine derartige Ausgestaltung der Basiszone nahezu über die gesamte Fußbreite eine erhebliche Verbesserung des Tragekomforts erzielt, da der Fuß in seiner gesamten Breite auf der Basisstruktur aufliegt und somit keine unangenehmen Stufen oder Ränder für den Patienten bemerkbar sind.

Bei einer vorteilhaften Weiterbildung der Erfindung ist zusätzlich oder alternativ vorgesehen, dass sich die Basisstruktur auch in Längsrichtung des Fußes gesehen bei bestimmungsgemäßem Gebrauch zumindest unterhalb der Großzehe erstreckt. Die Ausdehnung der Basisstruktur kann somit insbesondere in Abhängigkeit von der Fußform, dem Einsatzzweck bzw. Einsatzort und weiteren Voraussetzungen ausgestaltet werden.

Die Basisstruktur erstreckt sich bei einigen vorteilhaften Weiterbildungen neben dem Bereich unterhalb der Großzehe auch teilweise oder vollständig in dem Bereich unterhalb der weiteren Zehen. Dies ist beispielsweise bei Ausführungsformen sinnvoll, bei denen die zweite Befestigungseinrichtung in diesem Bereich auf der Unterseite der Basisstruktur fixiert wird. Doch auch bei anderen Ausführungsformen der erfindungsgemäßen Vorrichtung kann diese Ausbildung der Basisstruktur sinnvoll sein, insbesondere da die Zehen somit alle auf einer gemeinsamen Fläche stehen, was den Tragekomfort erhöht.

Des Weiteren kann in einer vorteilhaften Ausführungsform die Basisstruktur in Längsrichtung des Fußes gesehen derart ausgedehnt ausgebildet sein, dass sämtliche Zehen im bestimmungsgemäßen Gebrauch der Korrekturvorrichtung vollständig auf der Basisstruktur aufliegen können.

Damit die erfindungsgemäße Vorrichtung noch bequemer z. B. in einem Schuh und somit auch im Alltag tragbar ist, ist bei einer vorteilhaften Weiterbildung der erfindungsgemäßen Vorrichtung die Basisstruktur im Wesentlichen flachplattenförmig ausgebildet und besitzt einen begrenzten Bereich, der als elastisch in Flexions-Extensionsrichtung biegbare Biegezone ausgebildet ist oder bildet alternativ selbst als Ganzes die Biegezone aus. Die Biegezone ist dabei so ausgebildet und angeordnet, dass der Halteschenkel und der Korrekturschenkel bei bestimmungsgemäßem Gebrauch in einer natürlichen Beugerichtung der Zehen (Flexions-Extensionsrichtung) relativ zum Mittelfuß zueinander elastisch und/oder gelenkartig biegbar sind. Der Begriff flachplattenförmig ist so zu verstehen, dass die Basisstruktur eine im Wesentlichen flächige Ausdehnung aufweist. Dabei ist es jedoch nicht zwingend erforderlich, dass die Basisstruktur eben ist. Die flächige Ausdehnung trägt in besonderem Maße dazu bei, dass die Korrekturkraft, die von dem Korrekturschenkel auf die zu korrigierende Zehe aufgebracht wird, zu einem wesentlichen Teil durch die Steifigkeit der erfindungsgemäßen Vorrichtung und insbesondere durch die Steifigkeit der Basisstruktur aufgenommen wird.

Mit einer derartigen Biegezone ist es möglich, auch wenn die erfindungsgemäße Vorrichtung bestimmungsgemäß an dem Fuß befestigt ist, die Zehen auf natürliche Art und Weise abzuknicken bzw. zu beugen. Dies wird vorzugsweise dadurch erreicht, dass die Biegezone bei bestimmungsgemäßem Gebrauch in einem Bereich unterhalb zumindest eines Zehengrundgelenks angeordnet ist. Durch den Verzicht auf ein Gelenk oder Scharnier mit definierten Gelenk- oder Scharnierachsen und anstelle dessen die Verwendung der erfindungsgemäßen elastischen Biegezone findet auch bei einer Anordnung bei bestimmungsgemäßem Gebrauch unterhalb des Fußes eine gute Anpassung der erfindungsgemäßen Vorrichtung an die Form des Fußes und/oder der Zehen statt, was ebenfalls zu einem hohen Tragekomfort beiträgt.

Erfindungsgemäß gibt es mehrere Möglichkeiten die Biegezone auszubilden. Bei einer vorteilhaften Weiterbildung der Erfindung ist vorgesehen, dass die Biegezone nach Art eines Filmscharniers oder Bandscharniers gebildet ist. Das bedeutet, dass die Basisstruktur zumindest bereichsweise eine verringerte Materialstärke oder Dicke aufweist, wobei die Materialstärke oder Dicke der Basisstruktur in der Biegezone verringert ist, vorzugsweise mehr als 50%. Vorteilhafter Weise wird die Reduzierung der Materialstärke oder Dicke der Basisstruktur in der Biegezone auf der bei bestimmungsgemäßem Gebrauch unteren Seite der Basisstruktur vorgenommen. Auf diese Weise entsteht auf der Seite der Basisstruktur, auf der der Fuß bei bestimmungsgemäßem Gebrauch liegt, keine den Tragekomfort beeinträchtigende Kante oder Stufe. Durch die geringere Materialstärke bzw. Dicke im Bereich der Biegezone ist die Basisstruktur in diesem Bereich elastischer und lässt sich dort in Flexions-/Extensionsrichtung leichter verbiegen. Das Filmscharnier muss dabei nicht zwingenderweise in Form einer exakten, insbesondere geraden Schwenkachse ausgebildet sein. Erfindungsgemäß wird es viel mehr als vorteilhaft angesehen, wenn das Filmscharnier zum einen dem Verlauf der Großzehengrundgelenke in Fußquerrichtung, die in der Regel nicht entlang einer Geraden angeordnet sind, angepasst ist und zum anderen eine gewisse Breite, also eine signifikante Ausdehnung in Fußlängsrichtung nach Art eines Bandscharniers aufweist, so dass das Filmscharnier eine flächige Erstreckung unterhalb der Großzehengrundgelenke aufweist.

Alternativ oder zusätzlich kann die erfindungsgemäße Vorrichtung aus zwei verschiedenen Komponenten, einer ersten Komponente und einer zweiten Komponente, bestehen. Auf diese Weise lassen sich in unterschiedlichen Bereichen der erfindungsgemäßen Vorrichtung unterschiedliche Materialeigenschaften realisieren. Vorteilhafterweise besteht die erfindungsgemäße Vorrichtung aus Kunststoff, wobei dann vorzugsweise die erste Komponente ein erster Kunststoffwerkstoff und die zweite Komponente ein zweiter Kunststoffwerkstoff ist. Bei einer Ausführungsform der erfindungsgemäßen Vorrichtung ist dabei die erste Komponente härter als die zweite Komponente.

Als besonders vorteilhaft wird eine Ausführungsform der erfindungsgemäßen Vorrichtung angesehen, bei der die zweite Komponente elastisch reversibel biegbar ist.

In Bezug auf die Biegezone führt dies dazu, dass bei einer vorteilhaften Weiterbildung der erfindungsgemäßen Vorrichtung der Halteschenkel und der Korrekturschenkel aus der ersten Komponente und zumindest die Biegezone aus der zweiten Komponente gebildet werden.

Bzgl. der Auswahl der Komponenten stehen erfindungsgemäß mehrere Möglichkeiten zur Verfügung.

Da es insbesondere bei Kunststoff möglich ist, die Materialeigenschaften eines Materials durch Hinzugabe von Zusatzstoffen zu beeinflussen, besteht grundsätzlich die Möglichkeit, dass beide Komponenten im Wesentlichen aus dem chemisch gleichen Stoff bestehen und sich nur im Hinblick auf die Zusatzstoffe unterscheiden. Dies wird beispielhaft anhand Polyvinylchlorids (PVC) erläutert. Dieser Kunststoff zeichnet sich nämlich insbesondere dadurch aus, dass durch Hinzufügen von Weichmachern die Härte und die Zähigkeit von PVC verändert werden kann. So ist es beispielsweise möglich, dass die Zugfestigkeit von sogenanntem Hart-PVC das 5-fache der Zugfestigkeit von sogenanntem Weich-PVC beträgt. In diesem Fall wäre es also beispielsweise möglich, Hart-PVC als erste Komponente und Weich-PVC als zweite Komponente auszuwählen.

Alternativ ist es jedoch auch möglich, dass es sich bei der ersten Komponente und bei der zweiten Komponente um zwei unterschiedliche Stoffe handelt. Dabei wird für die erste Komponente vorzugsweise Polyethylen (PE), Polypropylen (PP), das bereits genannte Hart-PVC, Polyurethan (PU) sowie faserverstärkter Kunststoff, insbesondere kohlefaserverstärkter Kunststoff (CFK), glasfaserverstärkter Kunststoff (GFK) und aramidfaserverstärkter Kunststoff verwendet. Als zweite Komponente kommen erfindungsgemäß vorzugsweise Polyethylen (PE), Weich-PVC, Polyethylenterephthalat (PET) sowie ebenfalls Polyurethan zum Einsatz.

Bei einer vorteilhaften Weiterbildung der erfindungsgemäßen Vorrichtung ist die Basisstruktur mittels einer ersten Übergangszone mit dem Halteschenkel und/oder mittels einer zweiten Übergangszone mit dem Korrekturschenkel verbunden. Diese Übergangszonen ermöglichen eine stabile Verbindung der Basisstruktur mit dem Halteschenkel und/oder dem Korrekturschenkel, was insbesondere im Zusammenhang mit der einstückigen Ausführung der erfindungsgemäßen Vorrichtung zu deren Stabilität und Langlebigkeit beiträgt. Vorzugsweise ist die erste Übergangszone und/oder die zweite Übergangszone der Form des bei bestimmungsgemäßem Gebrauch anliegenden Bereichs des Fußes angepasst, so dass sich die erfindungsgemäße Vorrichtung in diesen Übergangszonen an den Fuß anschmiegt. Dadurch wird der Tragekomfort in einem Schuh zusätzlich verbessert, da die erfindungsgemäße Vorrichtung, wenn sie, wie das vorzugsweise der Fall ist, mit einer entsprechend geringen Materialstärke oder Dicke ausgeführt wird, für den Anwender beim Tragen kaum wahrnehmbar ist. Dabei wird es als besonders vorteilhaft angesehen, wenn auch die weiteren Bestandteile der erfindungsgemäßen Vorrichtung an die Fußform oder die Fußkontur angepasst sind.

In bestimmten Fällen kann es erforderlich sein, dass die erfindungsgemäße Vorrichtung, wie das bei einer vorteilhaften Weiterbildung der Fall ist, derart ausgebildet ist, dass der Korrekturschenkel mittels einer zweiten Befestigungseinrichtung an der zu korrigierenden Zehe befestigbar ist. Eine derartige zweite Befestigungseinrichtung ermöglicht es, dass der Korrekturschenkel lediglich mittelbar auf die zu korrigierende Großzehe einwirkt, was insbesondere beim Vorhandensein von weiteren physischen Symptomen an dem betroffenen Fuß von Vorteil sein kann. Zu diesen physischen Symptomen zählen beispielsweise ekzematische oder hautempfindliche OP-Narben, insbesondere solche, die von einer Operation bezüglich der zu korrigierenden Zehe herrühren. Problematisch können auch außergewöhnlich kurze Zehen sein, da in einem solchen Fall die Anordnung des Korrekturschenkels zwischen der Großzehe und der danebenliegenden zweiten Zehe als sehr unangenehm empfunden werden kann. Auch in diesem Fall kann es sinnvoll sein, den Korrekturschenkel im fußinnenseitigen Bereich der Großzehe anzuordnen.

Die zweite Befestigungseinrichtung ist vorzugsweise eine Ringbandage. Gleiches gilt vorzugsweise auch für die erste Befestigungseinrichtung. Dabei werden Ringbandagen aus Gewebeband oder einem, insbesondere hinsichtlich des Tragekomforts, vergleichbaren Material als besonders vorteilhaft angesehen.

Bei einer vorteilhaften Weiterbildung der erfindungsgemäßen Vorrichtung weisen der Halteschenkel und/oder der Korrekturschenkel schlitzförmige Ausnehmungen auf, mittels derer die erste Befestigungseinrichtung mit dem Halteschenkel und/oder die zweite Befestigungseinrichtung mit dem Korrekturschenkel verbunden ist.

Diese Ausnehmungen werden vorzugsweise in Kombination mit Ringbandagen eingesetzt. Auf diese Weise wird ein einfaches Fixieren aber auch ein einfaches Abnehmen der erfindungsgemäßen Vorrichtung ermöglicht. Insbesondere kann die Ringbandage in einem Endbereich ein Klettelement aufweisen, welches im Zusammenspiel mit dem Material der Ringbandagen ein sicheres und einfach handzuhabendes Fixieren der erfindungsgemäßen Vorrichtung ermöglicht.

Vorteilhafterweise ist die Ringbandage zirkulär zugstarr, also nicht aus einem gummiartigen dehnbaren Material ausgebildet. Auf diese Art und Weise wird durch die Fixierung der erfindungsgemäßen Vorrichtung mittels der Ringbandage im Bereich des Mittelfußes ein Aufrichten des Fußquergewölbes gefördert. Es hat sich gezeigt, dass das Aufrichten des Fußquergewölbes im Zusammenspiel mit dem Aufbringen einer Korrekturkraft auf eine fehlgestellte Zehe zu einem besonders positiven Ergebnis führt. Die erfindungsgemäße Vorrichtung wirkt somit in zweierlei Art und Weise auf den Fuß ein.

Bei einer besonders vorteilhaften Weiterbildung der erfindungsgemäßen Vorrichtung setzt sich der Randkonturverlauf aus mehreren Abschnitten zusammen. Demnach folgt der Randkonturverlauf zunächst einer im bestimmungsgemäßen Gebrauch oben liegenden Kante des Halteschenkels und geht dann in einen ersten Übergangsabschnitt über, wobei sich der Randkonturverlauf dabei der Basisstruktur annähert. Der erste Übergangsabschnitt liegt bei bestimmungsgemäßem Gebrauch in einem im Vergleich zu dem Seitenbereich, in dem sich eine Pseudoexostose bilden kann, proximalen ersten Abschnittsbereich. Im allgemeinen Verständnis liegt der erste Übergangsabschnitt also in Fußlängsrichtung gesehen "hinter" diesem Seitenbereich. Auf diese Weise wird sichergestellt, dass der Randkonturverlauf den Seitenbereich frei lässt.

Von dem ersten Übergangsabschnitt geht der Randkonturverlauf dann in einen Grundabschnitt über, der vorzugsweise im Wesentlichen U-förmig ist. Die U-Form des Grundabschnitts ist dabei nicht zwingend erforderlich. Der Grundabschnitt kann vielmehr auch andere, insbesondere andere kurvenförmige Konturen aufweisen, z. B. den kurvenförmigen Abschnitt der Außenkontur eines Kreis- oder eines Ellipsensegments. Vorzugsweise wird der Randkonturverlauf im Grundabschnitt insbesondere so gewählt, dass der Bereich, in dem sich eine Pseudoexostose bilden kann, freigelassen wird. Dabei wird es als besonders vorteilhaft angesehen, wenn der Randkonturverlauf der erfindungsgemäßen Vorrichtung im Bereich des Grundabschnitts, insbesondere im vertikal untersten Bereich bis auf das Niveau der Basisstruktur reicht. Dabei kann der Randkonturverlauf in einer Seitenansicht auf die erfindungsgemäße Vorrichtung, besonders bevorzugt tangential in die Basisstruktur einlaufen oder ausgehend von dem Halteschenkel in einem Winkel β bzw. ausgehend von dem Korrekturschenkel in einem Winkel γ auf diese treffen. Die Winkel β und/oder γ können sinnvoller Weise in einem Bereich zwischen 5° ≤ β, γ ≤ 45° liegen. Auf diese Weise wird jeglicher Druck auf etwaige Pseudoexostosen verhindert. Dabei ist es selbstverständlich, dass sich der Winkel ß und der Winkel γ nicht entsprechen müssen, d. h. gleich groß sein, sondern dass diese, je nach den gegebenen Voraussetzungen, wie beispielsweise der Fußform oder bereits bestehenden Pseudoexostosen, an dem zu behandelnden Fuß entsprechend angepasst werden können.

Es ist zudem möglich, den Randkonturverlauf seitens des Halteschenkels und seitens des Korrekturschenkels unterschiedlich zu gestalten. So ist es möglich, den Randkonturverlauf einerseits tangential in die Basisstruktur einlaufen zu lassen, während der Randkonturverlauf auf der anderen Seite in einem Winkel auf die Basisstruktur bzw. den Grundabschnitt trifft.

Ein Spezialfall liegt vor, wenn der Randkonturverlauf sowohl ausgehend von dem Halteschenkel als auch ausgehend von dem Korrekturschenkel tangential in dem gleichen Punkt (in der Seitenansicht gesehen) in die Basisstruktur einläuft. In diesem Fall beschränkt sich die Biegezone zumindest in den der Fußinnenseite nahe liegenden Bereichen im Wesentlichen auf eine Biegeachse. Liegen die beiden Punkte, an denen der Randkonturverlauf in die Basisstruktur einläuft hingegen eine Strecke > 0 (gemessen entlang des Randkonturverlaufs) auseinander, so ergibt sich eine Biegezone. Um sicherzustellen, dass der Seitenbereich, in dem sich eine Pseudoexostose bilden kann, frei bleibt, beträgt die Strecke zwischen diesen beiden Punkten bevorzugt mindestens 10 mm, vorzugsweise mehr als 20 mm.

Vorteilhafterweise geht der Randkonturverlauf aus dem Grundabschnitt in einem zweiten Übergangsabschnitt über, in dem sich der Randkonturverlauf ausgehend von dem Grundabschnitt von der Basisstruktur entfernt. Der zweite Übergangsabschnitt liegt bei bestimmungsgemäßem Gebrauch in einem im Vergleich zu dem Seitenbereich distaleren zweiten Abschnittsbereich, nach dem allgemeinen Verständnis also in Fußlängsrichtung gesehen "vor" dem Seitenbereich. So wird ebenfalls sichergestellt, dass der Randkonturverlauf den Seitenbereich freilässt. Aus dem zweiten Übergangsabschnitt geht der Randkonturverlauf dann in die Kontur einer bei bestimmungsgemäßen Gebrauch oben liegenden Kante des Korrekturschenkels über. Insgesamt ergibt sich somit ein Teil des Randkonturverlaufs, der sich, ausgehend von dem Halteschenkel, der Basisstruktur annähert, mögliche Pseudoexostosen möglichst vollständig umgeht und sich dann wieder von der Basisstruktur zu dem Korrekturschenkel hin entfernt.

Der Randkonturverlauf ist jedoch nicht auf diese Abschnitte beschränkt. So kann es aufgrund der Anforderungen an die erfindungsgemäße Vorrichtung erforderlich sein, dass weitere Abschnitte vorgesehen werden. So sind bei einer vorteilhaften Weiterbildung der erfindungsgemäßen Vorrichtung ein erster Zwischenabschnitt und ein zweiter Zwischenabschnitt vorgesehen, wobei der erste Zwischenabschnitt zwischen dem ersten Übergangsabschnitt und dem Grundabschnitt liegend und der zweite Zwischenabschnitt zwischen dem zweiten Übergangsabschnitt und dem Grundabschnitt liegend angeordnet sind.

Vorteilhafter Weise verläuft der Randkonturverlauf in dem ersten und dem zweiten Zwischenabschnitt jeweils entlang einer Geraden. Auf diese Weise ist es möglich, einen definierten Bereich zu schaffen, der von dem Randkonturverlauf freigelassen wird. Hierzu ist bei einer Weiterbildung der erfindungsgemäßen Vorrichtung vorgesehen, dass die Geraden, entlang derer der Randkonturverlauf in dem ersten und dem zweiten Zwischenabschnitt verläuft, in einem bestimmten Öffnungswinkel zueinander angeordnet sind. Dieser Öffnungswinkel beträgt zwischen 50° und 160°, vorzugsweise zwischen 70° und 130°, insbesondere etwa 90°.

Als besonders vorteilhaft wird ein Öffnungswinkel von mind. 90° angesehen, da eine natürliche Beugung der Zehen von bis zu 90° oder sogar darüber hinaus möglich ist. Bei einem Öffnungswinkel von 90° besteht somit keine Gefahr eines Einklemmens von Haut, da der von dem Randkonturverlauf freigelassene Seitenbereich groß genug ist.

Eine weitere Möglichkeit, um sicher zu stellen, dass der vom Randkonturverlauf freigelassene Bereich groß genug ist, um den Gelenkbereich der Fußinnenseite oder etwaige Pseudoexostosen vor Druck zu schützen, besteht darin, den Grundabschnitt derart auszuführen, dass er teilweise einen Radius von zumindest 5 mm, vorzugsweise zumindest 10 mm, insbesondere zumindest 15 mm aufweist. Besonders vorteilhaft ist eine derartige Ausführung des Grundabschnitts zusammen mit dem zuvor genannten Öffnungswinkel bzgl. des ersten und des zweiten Zwischenabschnitts.

Bei einer besonders vorteilhaften Weiterbildung ist der Korrekturschenkel der erfindungsgemäßen Vorrichtung zwischen der Großzehe und der daneben liegenden zweiten Zehe angeordnet. Der Korrekturschenkel ist dabei als von der Basisstruktur nach oben abgehender Steg ausgebildet. Mit einem derartig ausgestalteten Korrekturschenkel ist es möglich, auf die zweite Befestigungseinrichtung zu verzichten, da der Korrekturschenkel die Großzehe nunmehr in Richtung der Fußinnenseite drückt, also unmittelbar auf die Großzehe einwirkt. Bei der vorbeschriebenen und teilweise auch bei bekannten Schienen wird mit einer Bandage die Zehe in die gewollte Richtung gezogen, weshalb bei derartigen Schienen eine zweite Befestigungseinrichtung an dem Korrekturschenkel nötig ist. Derartige Befestigungseinrichtungen im Bereich der Zehen werden jedoch manchmal als unangenehm empfunden, da diese im Schuh einen erhöhten Platzbedarf benötigen. Damit die Schiene beim in den Schuh schlüpfen und beim Tragen nicht stört ist daher wünschenswert, diese Befestigungseinrichtungen, wenn möglich, zu vermeiden. Nichts desto trotz kann es in Einzelfällen nötig sein, auch einen zwischen der Großzehe und der daneben liegenden Zehe befindlichen Korrekturschenkel an der Großzehe mittels einer Befestigungseinrichtung zu fixieren, was jedoch auch mit der beschriebenen Weiterbildung der erfindungsgemäßen Vorrichtung ohne Weiteres möglich ist.

Bei einem sehr fortgeschrittenen Hallux-Valgus ist es auch möglich, dass nicht nur die Großzehe sondern die auch die danebenliegende zweite Zehe, aus ihrer natürlichen Position heraus in Richtung der restlichen Zehen und somit in Richtung der Fußaußenseite, fehlgestellt ist oder in anderen Fehlstellungen, wie z.B. der sogenannten Hammerzehe, verharren. Beim Hallux-Valgus ist es üblich, dass auch andere Zehen fehlgestellt sind, insbesondere aufgrund von zu geringem Platz im Schuh. Auch für derartige Fälle eignet sich die erfindungsgemäße Vorrichtung. Dabei ist es möglich, den Korrekturschenkel zwischen der zweiten und der in fußaußenseitiger Richtung von dieser liegenden dritten Zehe anzuordnen, wodurch bei bestimmungsgemäßem Gebrauch sowohl die zweite Zehe als auch die Großzehe in Richtung ihrer natürlichen Position gedrückt werden können. Ebenso ist hier auch die Verwendung der zweiten Befestigungseinrichtung in diesem Zusammenhang möglich, wobei dieser dann entweder an der zweiten Zehe oder sogar der Großzehe und der zweiten Zehe befestigt wird. Auf diese Weise kann durch die zweite Befestigungseinrichtung oder auch eine dritte, vierte oder weitere Befestigungseinrichtung, die an einem oder mehreren der Zehen befestigt sind, eine weitere, in Richtung der Basisstruktur, also plantar wirkende Korrekturkraft auf zu korrigierende Zehen eingebracht werden, beispielsweise auf Hammerzehen. Als Hammerzehen bezeichnete Zehen weisen ein Krankheitsbild auf, welches oftmals, aber nicht ausschließlich im Zusammenhang mit einer Hallux-Valgus-Erkrankung auftritt. Hammerzehen sind bogenartig gekrümmt und somit in einer Draufsicht auf den Fuß kürzer als eine gesunde Zehe.

Bei Ausführungsformen der erfindungsgemäßen Vorrichtung, die eine Basisstruktur aufweisen, die sich zumindest teilweise in einem Bereich unterhalb einer der Zehen erstreckt, kann mittels einer oder mehrerer der zweiten, dritten oder weiteren Befestigungseinrichtung eine Korrekturmöglichkeit für Hammerzehen geschaffen werden.

Durch die erfindungsgemäße Vorrichtung ist es somit möglich, bei Bedarf zwei Arten von Zehenfehlstellungen gleichzeitig zu behandeln. Mittels der zweiten Befestigungseinrichtung kann die durch die Hallux-Valgus-Erkrankung fehlgestellte Zehe in die ortho-gerade, also die natürliche, Stellung bewegt werden. Gleichzeitig ist es möglich durch weitere (dritte, vierte, fünfte) Befestigungseinrichtungen z.B. auf Hammerzehen therapierend einzuwirken, indem diese von einer weiteren Befestigungseinrichtung, insbesondere einer von dieser gebildeten Schlaufe, umfasst und in Richtung der Basisstruktur gezogen werden. Dadurch wird eine Streckung der Hammerzehen erwirkt und somit eine Korrekturwirkung erzielt. Eine Besonderheit dieser Ausführungsformen der erfindungsgemäßen Vorrichtung ist demnach die kombinatorische Wirkung auf die Hallux-Valgus-Fehlstellung und auf Hammerzehen.

Die weiteren (dritten, vierten, fünften) Befestigungseinrichtungen werden vorteilhafterweise auf gleiche Art an der Basisstruktur befestigt wie die zweite Befestigungseinrichtung. Auch sind die weiteren Befestigungseinrichtungen bevorzugt entsprechend der zweiten Befestigungseinrichtung, also vorzugsweise als Ringbandage, ausgebildet.

Es ist auch möglich die erfindungsgemäße Vorrichtung durch diverse Zubehöreinrichtungen zu ergänzen. Ein Beispiel hierfür ist eine sogenannte Spreizfußpelotte, die bei bestimmungsgemäßem Gebrauch der erfindungsgemäßen Vorrichtung unterhalb der Fußsohle in einem Bereich zwischen den Zehengrundgelenken und der Ferse angeordnet ist. Diese Spreizfußpelotte kann hierzu beispielsweise mittels einer Klettverbindung an der ersten Befestigungseinrichtung fixiert werden.

Des Weiteren kann die erfindungsgemäße Vorrichtung auch mit einer oder mehreren Polsterungen versehen werden, was den Tragekomfort der erfindungsgemäßen Vorrichtung weiter erhöht. Als besonders vorteilhaft werden Polsterungen insbesondere auf der Innenseite des Halteschenkels und/oder des Korrekturschenkels angesehen. Innenseite bedeutet in diesem Fall zum Fuß hin, also zwischen dem jeweiligen Schenkel und dem Fuß.

Bei vorteilhaften Weiterbildungen der erfindungsgemäßen Vorrichtung weist die erfindungsgemäße Vorrichtung eine Aufnahme für insbesondere optisch wirksame Zierelemente auf. Bekanntermaßen besteht bei vielen Menschen eine (natürliche) Abneigung gegenüber medizinischen Vorrichtungen wie Spritzen, Krücken oder Gipsverbänden. Diese Abneigung ist besonders ausgeprägt, wenn sich der Grund für die Verwendung der medizinischen Vorrichtung als Makel darstellt. Ein Fuß mit einer Hallux-Valgus-Fehlstellung wird oftmals als außerordentlicher Makel empfunden. Hinzu kommt, dass die Hallux-Valgus-Fehlstellung häufig bei Frauen vorkommt, die viel Wert auf ihre Erscheinung legen, da diese Fehlstellung unter anderem durch zu enge Schuhe begünstigt wird, die wiederum von Frauen oftmals gerne getragen werden. Durch die Möglichkeit, ein oder mehrere Zierelemente an der erfindungsgemäßen Vorrichtung zu fixieren, kann die Abneigung gegenüber der erfindungsgemäßen Vorrichtung deutlich vermindert werden. Prinzipiell sind die Zierelemente beispielsweise auch für die Wiedererkennung der eigenen erfindungsgemäßen Vorrichtung in einer Anhäufung von diesen von Vorteil.

Die Zierelemente können auf vielerlei Weise an der Aufnahme befestigt werden. Grundsätzlich sind hier Clips-Lösungen denkbar. Gleichsam ist es auch möglich, ein Zierelement in Form einer Spange auszubilden, die die Basisstruktur an z. B. mehreren Randbereichen umgreift und so an dieser fixiert wird. Besonders vorteilhaft sind die Zierelemente in einem Mittelbereich einer oder mehrerer Zehen angeordnet, da diese Mittelbereiche beim Stehen oder Laufen nicht belastet werden.

Die Aufnahme für das Zierelement kann auch auf der Oberseite des Korrekturschenkels angeordnet sein, sodass das Zierelement, beispielsweise in Form eines Edelsteins, auf einem zwischen der Großzehe und der zweiten Zehe hervorragenden Korrekturschenkel angeordnet ist.

Ein Verfahren zur Herstellung einer orthopädischen Vorrichtung umfasst im Wesentlichen zwei Schritte. In einem ersten Schritt wird ein Rohling der erfindungsgemäßen orthopädischen Vorrichtung hergestellt. Dieser Rohling weist dabei einen Randkonturverlauf auf, der beim Anlegen an einen zu behandelnden Fuß einen fußinnenseitigen Seitenbereich des Großzehengrundgelenks nicht oder nur teilweise freilässt. In einem zweiten Schritt wird dann durch Abtragen von Material in diesem Seitenbereich der Rohling an den zu behandelnden Fuß individuell angepasst. Dabei wird zumindest so viel Material abgetragen, dass der Rohling bei bestimmungsgemäßem Gebrauch der erfindungsgemäßen orthopädischen Vorrichtung den fußinnenseitigen Seitenbereich des Großzehengrundgelenks des zu behandelnden Fußes freilässt.

Der Vorteil des Verfahrens zum Herstellen einer orthopädischen Vorrichtung liegt darin, dass eine Massenfertigung bzw. industrielle Fertigung des Rohlings ermöglicht wird, was sich insbesondere kostensenkend auswirkt. Zum anderen führt das Verfahren dazu, dass durch die individuelle Anpassung des Rohlings an den zu behandelnden Fuß dessen Form und die vorliegende Erkrankung bzw. auch weitere Erkrankungen berücksichtigt werden können.

Vorteilhaft ist es dabei, wenn zusätzlich zu dem Abtragen von Material in dem fußinnenseitigen Seitenbereich auch Material an anderen Stellen des Rohlings abgetragen wird (falls dies für den zu behandelnden Fuß notwendig ist) und dass der Rohling zusätzlich durch plastische Verformung, insbesondere durch Verbiegen, noch weiter an den zu behandelnden Fuß angepasst wird. Somit ist es möglich, durch das erfindungsgemäße Verfahren zum Herstellen einer orthopädischen Vorrichtung eine derartige Vorrichtung zu erhalten, die optimal an die Form und die weiteren Eigenschaften des zu behandelnden Fußes angepasst ist.

Eine weitere Anpassung der erfindungsgemäßen Vorrichtung durch das Verfahren kann auch in zeitlichen Abständen während des Heilungsverlaufs stattfinden. Insbesondere kann eine plastische, insbesondere eine thermoplastische, Verformung mehrfach während des Heilungsverlaufs vorgenommen werden, wobei die erfindungsgemäße Vorrichtung und insbesondere der Korrekturschenkel nachjustiert, d.h. auf den aktuellen Zustand oder die aktuelle Ausprägung des Hallux-Valgus angepasst, werden können. Auf diese Weise hat die erfindungsgemäße Vorrichtung dauerhaft eine bessere Wirkung auf die vom Hallux-Valgus betroffene Zehe.

Bei vorteilhaften Weiterbildungen des Verfahrens wird die Anzahl der schlitzförmigen Öffnungen und/oder der Montageeinrichtungen sowie deren Anordnung individuell an den zu behandelnden Fuß sowie die Ausprägung der Hallux-Valgus-Fehlstellung angepasst. In diesen Fällen weist der Rohling zunächst keine Öffnungen oder Montageeinrichtungen auf. Diese werden erst im Laufe des Verfahrens zur Herstellung einer orthopädischen Vorrichtung im Rahmen der individuellen Anpassung in den Rohling eingebracht. Gleiches gilt auch für das Korrekturelement, welches ebenfalls im Rahmen des Verfahrens zur Herstellung einer orthopädischen Vorrichtung an den zu behandelnden Fuß sowie die Ausprägung der Hallux-Valgus-Fehlstellung angepasst werden kann.

Nachfolgend wird die Erfindung anhand der Zeichnung beispielhaft näher erläutert. Dabei zeigen:
- Figur 1:: eine perspektivische Ansicht auf eine erste Ausführungsform der erfindungsgemäßen Vorrichtung;
- Figur 2:: eine Draufsicht auf die erfindungsgemäße Vorrichtung der Figur 1;
- Figur 3:: eine weitere perspektivische Ansicht auf die erfindungsgemäße Vorrichtung der Figur 1;
- Figur 4a:: eine an einem Fuß befestigte Ausführungsform der erfindungsgemäßen Vorrichtung in einem entlasteten Zustand;
- Figur 4b:: die Ausführungsform der Figur 4a in einem gebogenen Zustand;
- Figur 5:: eine weitere Ausführungsform der erfindungsgemäßen Vorrichtung in einer Seitenansicht;
- Figur 5a:: das Detail A der Figur 5;
- Figur 6:: die Ausführungsform der Figur 5 in einer Draufsicht.
- Figur 7:: eine weitere Ausführungsform der erfindungsgemäßen Vorsicht in einer Seitenansicht;
- Figur 8:: eine weitere Ausführungsform der erfindungsgemäßen Vorrichtung bei bestimmungsgemäßem Gebrauch in einer Draufsicht;
- Figur 9:: eine weitere Ausführungsform der erfindungsgemäßen Vorrichtung in einer Draufsicht;
- Figur 10:: eine weitere Ausführungsform einer nicht erfindungsgemäßen Vorrichtung in einer Vorderansicht;
- Figur 11:: eine weitere Ausführungsform der erfindungsgemäßen Vorrichtung in einer Draufsicht;
- Figur 12a:: ein Detail der erfindungsgemäßen Vorrichtung in einer weiteren Ausführungsform in einer ersten Lage;
- Figur 12b:: ein Detail der erfindungsgemäßen Vorrichtung in einer weiteren Ausführungsform in einer zweiten Lage.

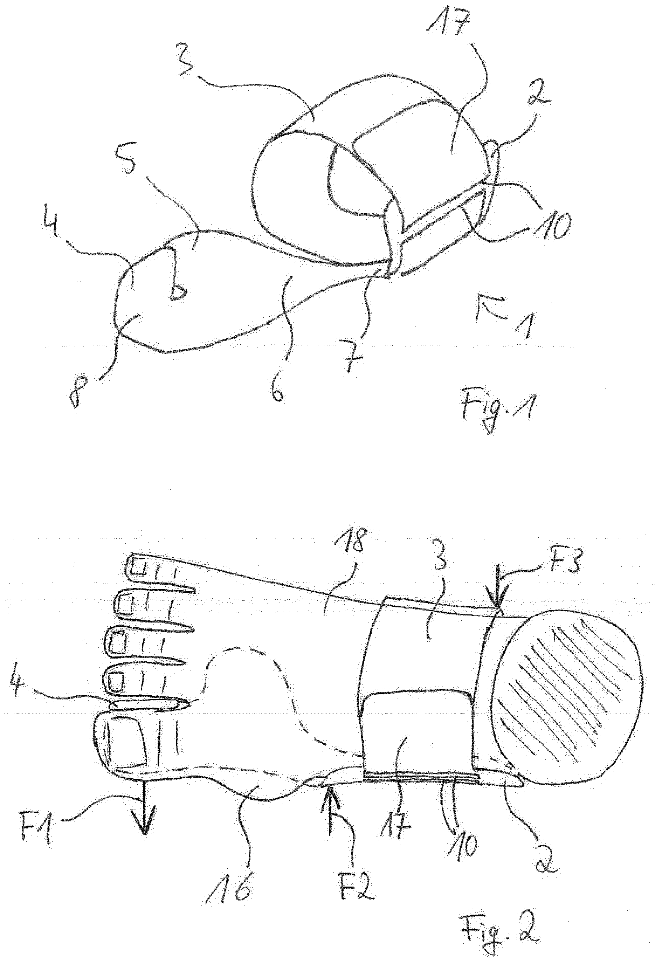

Die in der Figur 1 dargestellte Ausführungsform der erfindungsgemäßen Vorrichtung 1 weist einen Halteschenkel 2 auf, der bei bestimmungsgemäßem Gebrauch an einem fußinnenseitigen Bereich des Mittelfußes angeordnet ist. Der Halteschenkel 2 weist schlitzförmige Ausnehmungen 10 auf, mittels derer eine erste Befestigungseinrichtung 3 mit dem Halteschenkel 2 verbunden ist. Der Halteschenkel 2 ist in einer ersten Übergangszone 7 mit einer Basisstruktur 5 verbunden. Die Basisstruktur 5 ist flachplattenförmig ausgebildet und ist bei bestimmungsgemäßem Gebrauch unterhalb der Fußsohle des Fußes angeordnet.

Bei der in der Figur 1 gezeigten Ausführungsform besitzt die Basisstruktur 5 eine Ausdehnung, die sich über einen unter mehreren Zehengrundgelenken liegenden Bereich erstreckt. Es ist jedoch durch eine Strichlinie auch angedeutet, dass die Ausdehnung der Basisstruktur 5 beispielsweise auch nur den unter dem Großzehengrundgelenk liegenden Bereich umfassen kann (vgl. Fig. 2). In beiden Fällen umfasst aber die Basisstruktur 5 bei dieser Ausführungsform auch den unter der Großzehe liegenden Bereich.

Die erfindungsgemäße Vorrichtung 1 weist zudem einen Korrekturschenkel 4 auf, der in einer zweiten Übergangszone 8 mit der Basisstruktur 5 verbunden ist. Bei bestimmungsgemäßem Gebrauch ist der Korrekturschenkel 4 zwischen der Großzehe und der daneben liegenden, zweiten Zehe angeordnet und übt eine unmittelbar Korrekturkraft F1 in Form einer zur Fußinnenseite gerichteten Druckkraft auf die Großzehe aus, um die fehlgestellte Großzehe wieder in ihre natürliche Lage zurückzubewegen und so den Heilungsprozess zu unterstützen.

In den Bereichen der Basisstruktur 5, die bei bestimmungsgemäßem Gebrauch unterhalb von Zehengrundgelenken angeordnet sind, weist die Basisstruktur 5 eine Biegezone 6 auf. Durch diese Biegezone 6 ist es möglich, bei angelegter Schiene 1 die Zehen in der natürlichen Flexions-Extensionsrichtung zu bewegen, wodurch ein normales Laufen selbst dann möglich ist, wenn die erfindungsgemäße Vorrichtung 1 an dem Fuß befestigt ist.

Bei der in Figur 1 dargestellten Ausführungsform der erfindungsgemäßen Vorrichtung 1 sind der Halteschenkel 2, die Basisstruktur 5 und der Korrekturschenkel 4 einstückig ausgebildet und bestehen aus Kunststoff. Die erste Befestigungseinrichtung 3 ist eine Ringbandage aus einem Gewebeband, welches ein Klettelement 17 aufweist, mittels dessen die erfindungsgemäße Vorrichtung 1 im Bereich des Mittelfußes an dem Fuß befestigt werden kann.

Die Figur 2 zeigt die Ausführungsform der erfindungsgemäßen Vorrichtung 1 der Figur 1 bei bestimmungsgemäßem Gebrauch. Die Vorrichtung 1 ist dabei an einem Fuß 18 befestigt. Der Fuß weist eine ballenförmige Ausbuchtung (Pseudoexostose) in einem Seitenbereich 16 auf. Dieser Seitenbereich 16 wird von dem Randkonturverlauf der erfindungsgemäßen Vorrichtung 1 frei gelassen, so dass kein oder nur sehr wenig Druck auf die Pseudoexostose ausgeübt wird.

Die erfindungsgemäße Vorrichtung 1 ist mittels der ersten Befestigungseinrichtung 3 im Bereich des Mittelfußes an dem Fuß 18 befestigt und trägt so zu einer Aufrichtung des Mittelfußgewölbes bei. Der Korrekturschenkel 4 ist zwischen einer Großzehe 19 und einer zweiten Zehe 20 des Fußes 18 angeordnet und übt eine Korrekturkraft F1, die in die Richtung der Fußinnenseite wirkt, aus. Die Korrekturkraft F1 wird zum einen durch die Steifigkeit der Basisstruktur 5 und zum anderen durch die Befestigung der Vorrichtung 1 an dem Fuß 18 mittels des Halteschenkels 2 und der ersten Befestigungseinrichtung 3 kompensiert. Im Bereich der ersten Befestigungseinrichtung 3 wirken dadurch zwei Kräfte, eine erste Haltekraft F2 und eine zweite Haltekraft F3 auf den Fuß ein, da sich die Vorrichtung 1 durch die erste Befestigungseinrichtung 3 und den Halteschenkel 2 auf dem Fuß abstützt. In dem Seitenbereich 16 tritt jedoch keine Kraft auf, wie das bei herkömmlichen Schienen der Fall ist, so dass die erfindungsgemäße Vorrichtung 1 keinen Druck auf eine Pseudoexostose ausübt.

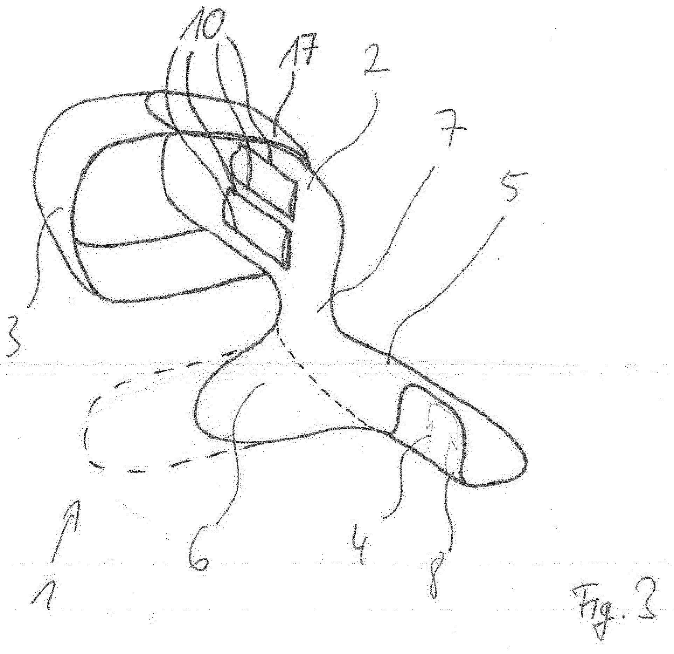

Die Figur 3 zeigt die Ausführungsform der erfindungsgemäßen Vorrichtung 1 der Figur 1 aus einer anderen Perspektive. Zu erkennen ist, dass der Halteschenkel 2 insgesamt vier schlitzförmige Ausnehmungen 10 aufweist, durch die die als Ringbandage ausgeführte erste Befestigungseinrichtung 3 geführt ist. Auch hier sind verschiedene Ausdehnungsmöglichkeiten der Basisstruktur 5 mittels Strichlinien dargestellt.

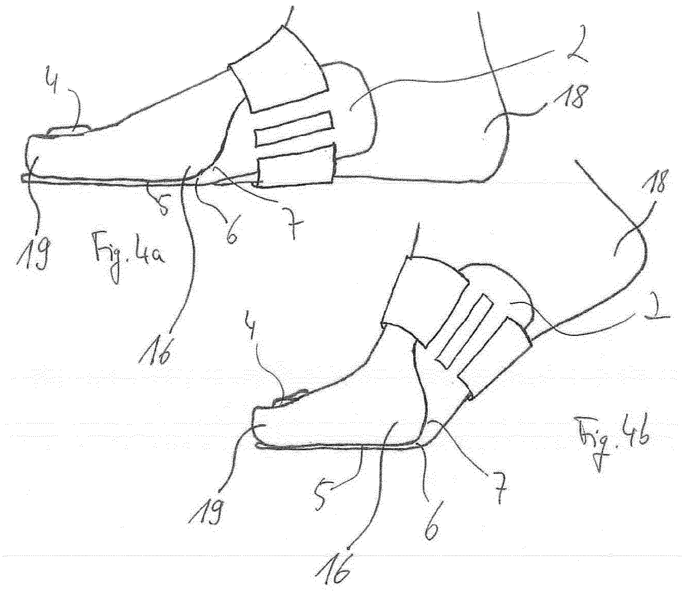

Die Figuren 4a und 4b zeigen die Funktionsweise der Biegezone 6 der erfindungsgemäßen Vorrichtung 1. Die Vorrichtung 1 ist an einem Fuß 18 befestigt, der sich in der Figur 4a im entspannten Zustand befindet. Im Falle der Figur 4b hat eine Beugung der Zehen stattgefunden. Das bedeutet, dass die Zehen eine Bewegung, wie sie beim normalen Gehen üblich ist, vollzogen haben. Es ist ersichtlich, dass, selbst bei einer derartigen Bewegung, der Seitenbereich 16, in dem eine Pseudoexostose vorliegen kann, frei bleibt. Auf diese Weise ist das Tragen der erfindungsgemäßen Vorrichtung 1 beim Gehen und somit im alltäglichen Gebrauch ohne ein unangenehmes Drücken in dem Seitenbereich 16 möglich.

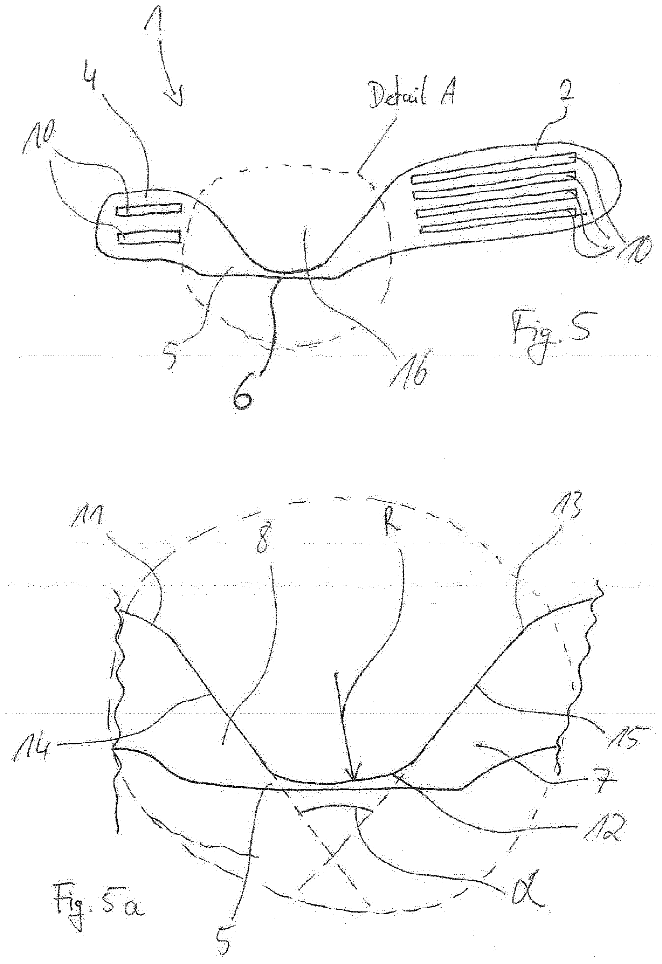

Die Figur 5 zeigt eine zweite Ausführungsform der erfindungsgemäßen Vorrichtung 1, die ebenfalls einen Halteschenkel 2, eine Basisstruktur 5 und eine Korrekturschenkel 4 aufweist. Bei dieser Ausführungsform sind sowohl der Halteschenkel 2 als auch der Korrekturschenkel 4, jeweils bei bestimmungsgemäßem Gebrauch, an einem fußinnenseitigen Bereich angeordnet. Damit der Korrekturschenkel 4 eine Korrekturkraft F1 auf die Großzehe ausüben kann, weist er zwei schlitzförmige Ausnehmungen 10 auf, durch die eine zweite Befestigungseinrichtung 9 (nicht dargestellt) geführt werden kann, mittels derer die Vorrichtung 1 an der Großzehe befestigt werden kann (mittelbare Einwirkung des Korrekturschenkels auf die Großzehe).

Die Biegezone 6 ist bei dieser Ausführungsform in der Nähe der Fußinnenseite lediglich als Biegeachse ausgebildet, da der Randkonturverlauf sowohl seitens des Halteschenkels 2 als auch seitens des Korrekturschenkels 4 tangential in die Basisstruktur 5 einläuft.

Auch bei dieser Ausführungsform wird ein Seitenbereich 16, in dem sich eine Pseudoexostose bilden kann bzw. gebildet hat, freigelassen.

Die Figur 5a zeigt das Detail A der Figur 5, in dem der Randkontorverlauf der erfindungsgemäßen Vorrichtung 1 zu erkennen ist. Ausgehend von einer bei bestimmungsgemäßem Gebrauch oben liegenden Kante des Halteschenkels 2 durchläuft der Randkonturverlauf einen ersten Übergangsabschnitt 13, in dem sich der Randkonturverlauf der Basisstruktur 5 annähert. Aus dem ersten Übergangsabschnitt 13 geht der Randkonturverlauf dann in einen ersten Zwischenabschnitt 15 über, der im Wesentlichen entlang einer Geraden verläuft und dann in einen Grundabschnitt 12 übergeht. Der Grundabschnitt 12 weist in einem Teilbereich die Form der gekrümmten Kante eines Kreisabschnitts mit einem Radius R auf. Der Randkonturverlauf geht aus dem Grundabschnitt 12 in einem zweiten Zwischenabschnitt 14 über, der ebenfalls entlang einer Geraden verläuft. Aus dem zweiten Zwischenabschnitt 14 geht der Randkonturverlauf in einen zweiten Übergangsabschnitt 11 und anschließend in eine, bei bestimmungsgemäßem Gebrauch, oben liegende Kante des Korrekturschenkels 4 über. Die Geraden, entlang derer der erste Zwischenabschnitt 15 und der zweite Zwischenabschnitt 14 verlaufen, weisen zueinander einen Öffnungswinkel α auf. Bei der dargestellten Ausführungsform der erfindungsgemäßen Vorrichtung 1 beträgt der Öffnungswinkel in etwa 90°.

Der erste Übergangsabschnitt 13 und der erste Zwischenabschnitt 15 liegen bei bestimmungsgemäßem Gebrauch in einem im Vergleich zu dem Seitenbereich 16 proximaleren ersten Abschnittsbereich 21 (siehe Figur 5). Der zweite Übergangsabschnitt 11 und der zweite Zwischenabschnitt 14 liegen bei bestimmungsgemäßem Gebrauch in einem im Vergleich zu dem Seitenbereich 16 distaleren zweiten Abschnittsbereich 22 (siehe Figur 5). Auf diese Weise verläuft der Randkonturverlauf derart, dass der Seitenbereich 16 frei gelassen wird.

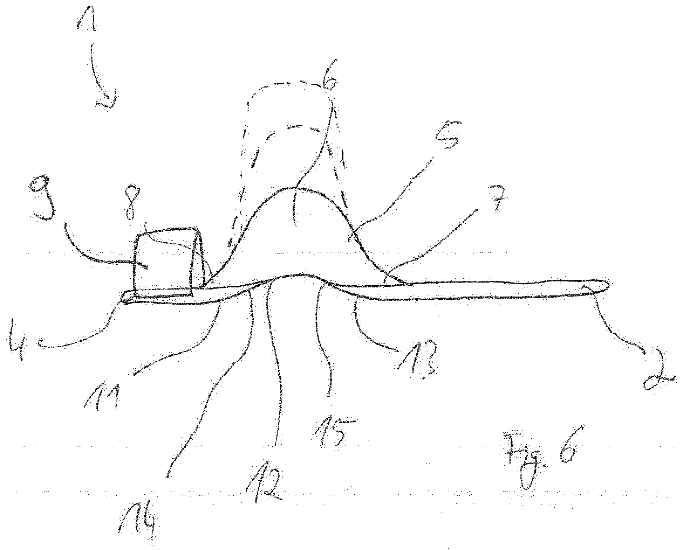

Die Figur 6 zeigt die in der Figur 5 dargestellte Ausführungsform der erfindungsgemäßen Vorrichtung 1 in einer Draufsicht. Die Basisstruktur 5 der dargestellten Ausführungsform weist eine Ausdehnung auf, die von den unter den Zehengrundgelenken liegenden Bereiche lediglich den Bereich umfasst, der unter dem Großzehengrundgelenk liegt. Auch bei dieser Ausführungsform ist es jedoch möglich, die Ausdehnung der Basisstruktur 5 anders zu gestalten, wie das beispielhaft mittels Strichlinien dargestellt ist.

Die in der Figur 6 dargestellte Ausführungsform ist wie die Ausführungsform der Figur 1 einstückig ausgebildet, wobei die Basisstruktur 5 auch hier in einer Übergangszone 7 mit dem Halteschenkel 2 und in einer zweiten Übergangszone 8 mit dem Korrekturschenkel 4 verbunden ist.

Ein wesentlicher Unterschied der in der Figur 6 dargestellten Ausführungsform ist, dass der Korrekturschenkel 4 auf der Fußinnenseite der Großzehe angeordnet ist. Aus diesem Grund ist bei dieser Ausführungsform eine zweite Befestigungseinrichtung 9 vorgesehen, mittels der der Korrekturschenkel 4 an der Großzehe befestigt wird und so eine Korrekturkraft auf diese ausüben kann.

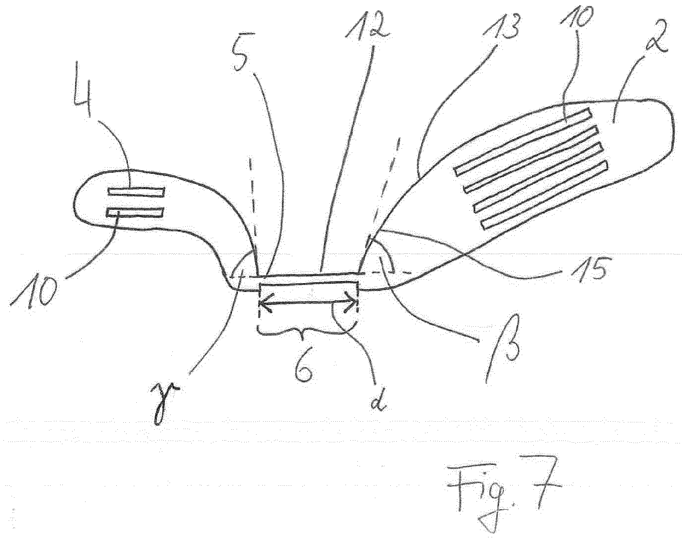

In der Figur 7 ist eine weitere Ausführungsform der erfindungsgemäßen Vorrichtung 1 dargestellt, deren Randkonturverlauf nicht tangential in die Basisstruktur 5 übergeht, sondern verglichen mit der Ausführungsform der Figur 5 eine relativ große Biegezone 6 ausbildet. Die Biegezone 6 erstreckt sich dabei entlang einer Strecke d des Randkonturverlaufs. Der Grundabschnitt 12, der in der Figur 7 dargestellten Ausführungsform der erfindungsgemäßen Vorrichtung 1 ist somit gerade. Von dem Halteschenkel 2 ausgehend geht der Randkonturverlauf aus dem ersten Übergangsabschnitt 13 in einen ersten Zwischenabschnitt 15 über und trifft dann in einem Winkel β auf den Grundabschnitt 12. Ebenso geht bei der gezeigten Ausführungsform der Randkonturverlauf ausgehend von dem Korrekturschenkel 4 aus einem zweiten Übergangsabschnitt 11 in einen zweiten Zwischenabschnitt 14 über und trifft dann in einem Winkel γ auf den Grundabschnitt 12.

Obgleich es bei den gezeigten Ausführungsformen jeweils der Fall ist, dass sich der Randkonturverlauf auf der Seite des Korrekturschenkels 4 und auf der des Halteschenkels 2 gleich verhält, also jeweils in einem Winkel auf den Grundabschnitt 12 trifft bzw. in beiden Fällen tangential in den Grundabschnitt übergeht, ist es ebenso möglich, dass diese Ausführungen des Randkonturverlaufs gemischt werden. So ist es beispielsweise möglich, dass ausgehend von dem Halteschenkel 2 der Randkonturverlauf in einem Winkel β auf den Grundabschnitt trifft und dass ausgehend von dem Korrekturschenkel 4 der Randkonturverlauf tangential in den Grundabschnitt 12 übergeht (nicht gezeigt).

Bei der in der Figur 7 dargestellten Ausführungsform ist die Dicke der Basisstruktur 5 im Bereich der Biegezone 6 verringert, was dazu beiträgt, dass die Basisstruktur 5 im Bereich der Biegezone 6 besonders gut elastisch in Flexions-Extensionsrichtung biegbar ist.

Der Korrekturschenkel 4 ist bei der in der Figur 7 dargestellten Ausführungsform ebenso wie der Halteschenkel 2 bei bestimmungsgemäßem Gebrauch an der Fußinnenseite angeordnet. Dies hat zur Folge, dass der Korrekturschenkel 4 ebenso wie der Halteschenkel 2 schlitzförmige Ausnehmungen 10 aufweist, mittels derer im Falle des Korrekturschenkels 4 eine zweite Befestigungseinrichtung 9 (nicht dargestellt) zur Befestigung der erfindungsgemäßen orthopädischen Vorrichtung 1 an dem zu korrigierenden Zehen befestigbar ist, mittels der schlitzförmigen Ausnehmungen 10 des Halteschenkels 2 kann dieser durch eine erste Befestigungseinrichtung 3 (nicht dargestellt) an dem Fuß im Bereich des Mittelfußes befestigt werden, wodurch die erfindungsgemäße orthopädische Vorrichtung zudem zum Aufrichten des Mittelfußgewölbes beiträgt.

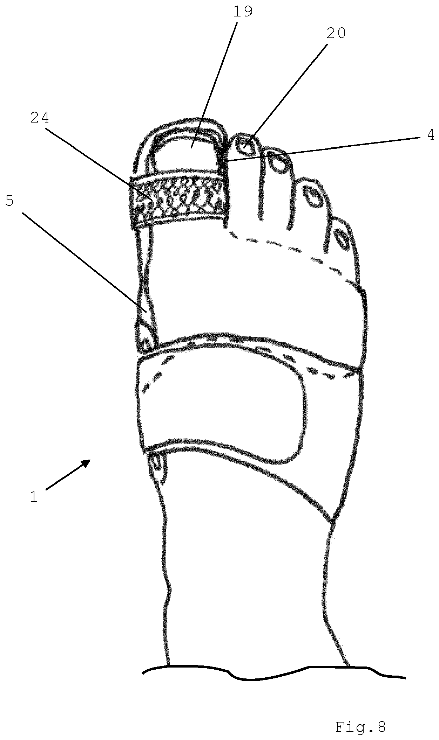

Die Figur 8 zeigt eine Ausführungsform der erfindungsgemäßen Vorrichtung 1 in einer Draufsicht und bei bestimmungsgemäßem Gebrauch, d.h. angelegt an einen zu behandelnden Fuß. Die Basisstruktur 5 erstreckt sich bei dieser Ausführungsform unterhalb sämtlicher Zehengrundgelenke. Der Korrekturschenkel 4 ist als Steg ausgebildet und zwischen der Großzehe 19 und der zweiten Zehe 20 angeordnet.

Die Ausführungsform der Figur 8 weist ein Zierelement 24 auf, welches als Spange ausgebildet ist. Das Zierelement 24 umgreift die Großzehe 19 in einem mittleren Bereich, der beim Laufen und/oder Stehen nicht oder kaum belastet wird, weshalb sich das Zierelement 24 nicht unangenehm anfühlt. Das Zierelement 24 ist mit einem Muster versehen, wodurch die erfindungsgemäße Vorrichtung 1 zum einen den medizinischen Charakter zu gewissem Maße verliert, da dieser zumindest teilweise durch einen modischen Charakter ersetzt wird. Zudem ist es dem Benutzer möglich, die eigene orthopädische Vorrichtung 1 alleine anhand des Zierelements 24 von anderen zu unterscheiden.

Das Zierelement 24 ist lösbar an der Basisstruktur 5 befestigt. Hierdurch ist es möglich, das Zierelement 24 beliebig oft durch ein anderes Zierelement auszutauschen, was eine individuelle Anpassung der orthopädischen Vorrichtung 1 beispielsweise an die Kleidung ermöglicht.

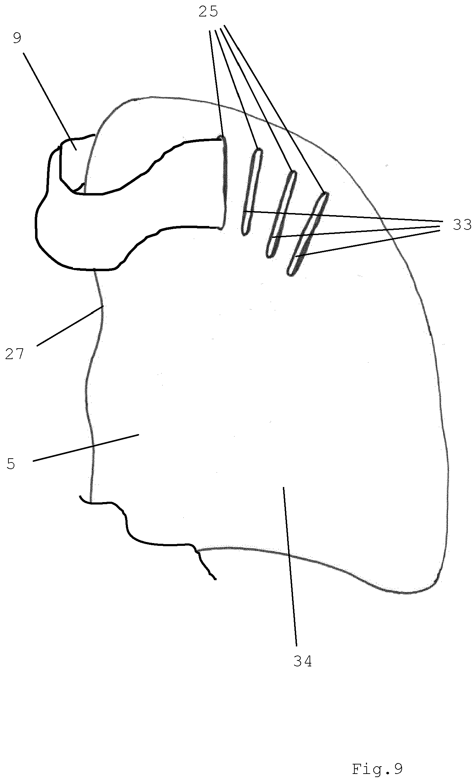

Die Figur 9 zeigt eine weitere Ausführungsform der erfindungsgemäßen Vorrichtung 1, bei der in der Basisstruktur 5 eine Mehrzahl von schlitzförmigen Öffnungen 25 vorgesehen ist. Eine zweite Befestigungseinrichtung 9 ist dabei durch eine der schlitzförmigen Öffnungen 25 geführt und legt die Großzehe 19 bei bestimmungsgemäßem Gebrauch der erfindungsgemäßen Vorrichtung hinsichtlich der Basisstruktur 5 fest. Je nach Ausprägung der Hallux-Valgus-Fehlstellung und der Fußform des zu behandelnden Fußes kann die zweite Befestigungseinrichtung 9 durch einen der schlitzförmigen Öffnungen 25 geführt und so angepasst werde.

Die zweite Befestigungseinrichtung 9 ist zu einem fußinnenseitigen Rand 27 der Basisstruktur 5 geführt und tritt dort von der Unterseite 26 der Basisstruktur 5, d. h. der Seite der Basisstruktur 5, auf der bei bestimmungsgemäßem Gebrauch der zu behandelnde Fuß aufliegt, auf deren Oberseite 34, d. h. der der Oberseite gegenüberliegenden Seite der Basisstruktur 5. Dort bildet die zweite Befestigungseinrichtung 9 eine Schlaufe, in der die Großzehe bei bestimmungsgemäßem Gebrauch angeordnet und somit bezüglich der Basisstruktur 5 festlegbar ist.

Die schlitzförmigen Öffnungen 25, durch welche die zweite Befestigungseinrichtung 9 nicht geführt wird, sind durch Einsteckelemente 33 verschlossen, sodass auf der Oberfläche der Basisstruktur 5 keine unangenehmen Kanten entstehen.

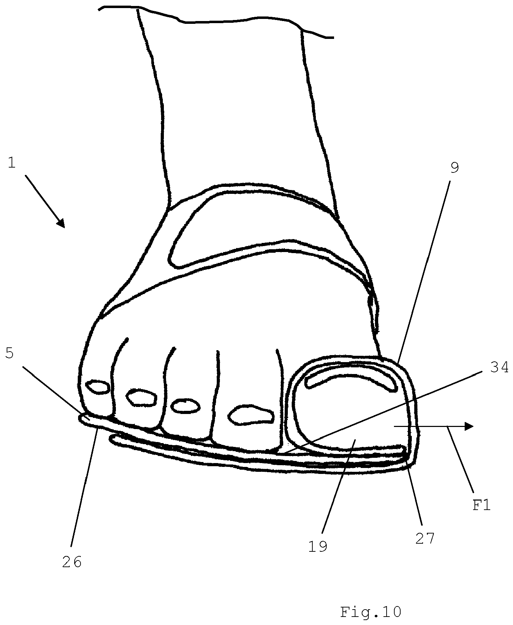

Die Figur 10 zeigt eine weitere Ausführungsform der erfindungsgemäßen Vorrichtung 1. Diese Ausführungsform weißt weder einen Korrekturschenkel noch schlitzförmige Öffnungen auf. Die Fixierung der Großzehe bezüglich der Basisstruktur 5 erfolgt durch eine zweite Befestigungseinrichtung 9, die auf der Unterseite 26 der Basisstruktur 5 fixiert ist. Von dort verläuft sie auf der Unterseite 26 der Basisstruktur 5 zu einem fußinnenseitigen Rand 27 der Basisstruktur 5. Dort bildet die zweite Befestigungseinrichtung 9 eine Schlaufe, die sich bei bestimmungsgemäßem Gebrauch um die Großzehe 19 legt, wodurch die Großzehe 19 mit einer Korrekturkraft F1 beaufschlagt wird, indem die Großzehe 19 in einer Position bezüglich der Basisstruktur 5 festgelegt wird, die der natürlichen Position der Großzehe 19 oder zumindest einer ähnlichen Position im Gegensatz zu der bei der Hallux-Valgus-Fehlstellung vorliegenden Position entspricht.

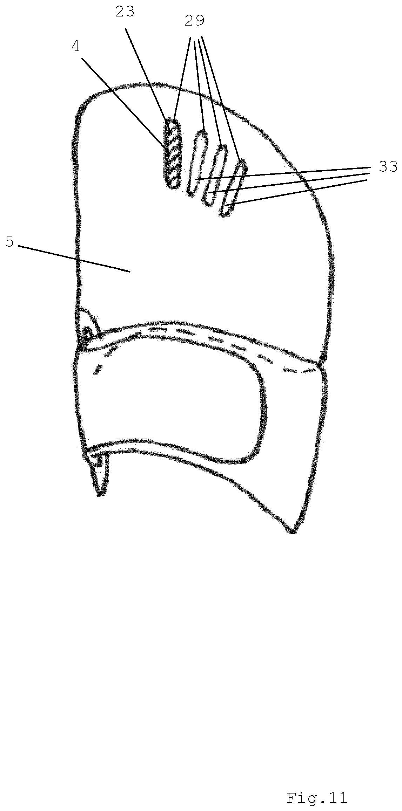

Die Figur 11 zeigt eine weitere Ausführungsform der erfindungsgemäßen Vorrichtung 1, bei der die Basisstruktur 5 eine Mehrzahl von Montageeinrichtungen 29 aufweist. In einer dieser Montageeinrichtungen 29 ist ein Korrekturelement 23 angeordnet, welches bei bestimmungsgemäßem Gebrauch als Korrekturschenkel 4 fungiert. Grundsätzlich ist jede Montageeinrichtung 29 so ausgestaltet, dass sie das Korrekturelement 23 aufnehmen und zu einem gewissen Grad fixieren kann.

Die Montageeinrichtungen 29, in denen kein Korrekturelement 23 angeordnet ist sind jeweils durch ein Einsteckelement 33 verschlossen, sodass keine unangenehmen Kanten an der Montageeinrichtung 29 entstehen.

Die Montageeinrichtungen 29 sind so angeordnet, dass sie der Abknickbewegung der Großzehe, die diese durch die Hallux-Valgus-Fehlstellung erfährt, folgen. Auf diese Weise ist eine Anpassung der orthopädischen Vorrichtung 1 an die Ausprägung der Hallux-Valgus-Fehlstellung und an die Fußform des zu behandelnden Fußes anpassbar.

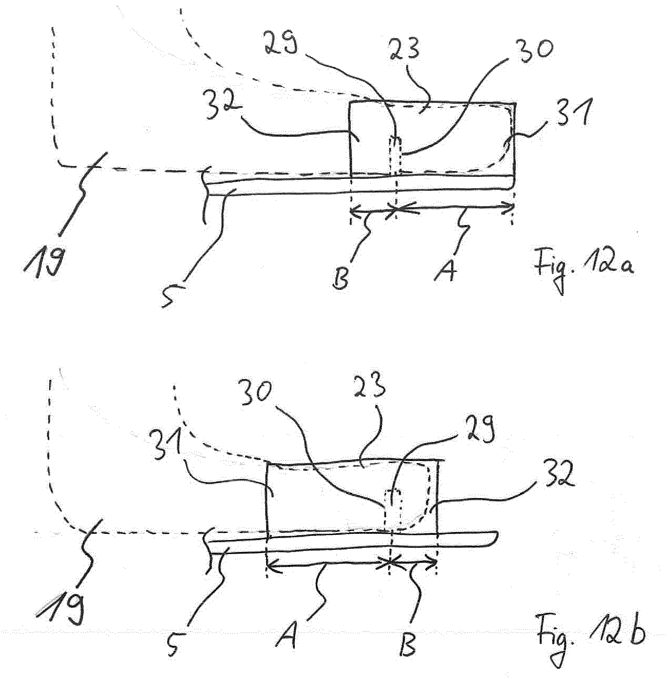

In den Figuren 12a und 12b ist ein Korrekturelement 23 dargestellt, welches mittels einer als Dorn ausgebildeten Montageeinrichtung 29 an der Basisstruktur 5 lösbar befestigt ist. Das Korrekturelement 23 weist eine Vertiefung 30 auf, die mit dem Dorn korrespondiert. Die Vertiefung 30 ist exzentrisch in dem Korrekturelement 23 vorgesehen, wodurch sie einen Abstand A zu einem ersten Rand 31 und einen Abstand B zu einem zweiten Rand 32 des Korrekturelements 23 aufweist. Der Abstand A ist dabei größer als der Abstand B.

Das Korrekturelement 23 kann in zwei Lagen auf der Montageeinrichtung 29 befestigt werden. In einer ersten Lage, die in Figur 12a dargestellt ist, weist der Abstand A in die Richtung, in die die Großzehe 19 weist. In einer zweiten Lage, die in Figur 12b dargestellt ist, weist der Abstand B in diese Richtung. Durch diese Lageverstellung kann die erfindungsgemäße Vorrichtung 1 an unterschiedlich lange Füße und/oder Zehen angepasst werden. Insbesondere kann die erfindungsgemäße Vorrichtung 1 so abgestimmt werden, dass das Korrekturelement 23 nicht bis in den Zehengrund reicht und diesen verletzt und/oder wund scheuert.

### Bezugszeichenliste

- 1: orthopädische Vorrichtung
- 2: Halteschenkel
- 3: erste Befestigungseinrichtung
- 4: Korrekturschenkel
- 5: Basisstruktur
- 6: Biegezone
- 7: erste Übergangszone
- 8: zweite Übergangszone
- 9: zweite Befestigungseinrichtung
- 10: Ausnehmung
- 11: zweiter Übergangsabschnitt
- 12: Grundabschnitt
- 13: erster Übergangsabschnitt
- 14: zweiter Zwischenabschnitt
- 15: erster Zwischenabschnitt
- 16: Seitenbereich
- 17: Klettelement
- 18: Fuß
- 19: Großzehe
- 20: zweite Zehe
- 21: erster Abschnittsbereich
- 22: zweiter Abschnittsbereich
- 23: Korrekturelement
- 24: Zierelement
- 25: schlitzförmige Öffnung
- 26: Unterseite
- 27: fußinnenseitiger Rand
- 29: Montageeinrichtung
- 30: Vertiefung
- 31: erster Rand
- 32: zweiter Rand
- 33: Einsteckelement
- 34: Oberseite

- α: Öffnungswinkel
- β: Winkel
- γ: Winkel
- R: Radius
- d: Strecke
- F1: Korrekturkraft
- F2: erste Haltekraft
- F2: zweite Haltekraft
- A: Abstand
- B: Abstand

## Patentansprüche

1. Orthopädische Vorrichtung, insbesondere zur Korrektur von Zehenfehlstellungen, mit einem Halteschenkel (2), einer ersten Befestigungseinrichtung (3), zumindest einem Korrekturschenkel (4) zum unmittelbaren oder mittelbaren Einwirken auf die Großzehe und einer den Halteschenkel (2) und den Korrekturschenkel (4) verbindenden plattenartigen Basisstruktur (5), wobei die Vorrichtung einen Randkonturverlauf aufweist, der bei bestimmungsgemäßem Gebrauch einen fußinnenseitigen Seitenbereich (16) des Großzehengrundgelenks freilässt, **dadurch gekennzeichnet, dass**
Alternative 1:
der Halteschenkel (2), der Korrekturschenkel (4) und die Basisstruktur (5) einstückig ausgebildet sind und sich die Basisstruktur (5) bei bestimmungsgemäßem Gebrauch zumindest unterhalb des Zehengrundgelenks der Großzehe erstreckt, wobei die Basisstruktur (5) eine Biegezone (6) besitzt oder bildet, wobei der Halteschenkel (2) und der Korrekturschenkel (4) bei bestimmungsgemäßem Gebrauch in einer natürlichen Beugerichtung der Zehen relativ zum Mittelfuß zueinander elastisch und/oder gelenkartig biegbar sind und wobei die Befestigungseinrichtung (3) zum Befestigen des Halteschenkels (2) im Bereich des Mittelfußes ausgebildet ist oder
Alternative 2:
sich die Basisstruktur (5) bei bestimmungsgemäßem Gebrauch zumindest unterhalb des Zehengrundgelenks der Großzehe erstreckt, wobei die Basisstruktur (5) eine Biegezone (6) besitzt oder bildet, wobei der Halteschenkel (2) und der Korrekturschenkel (4) bei bestimmungsgemäßem Gebrauch in einer natürlichen Beugerichtung der Zehen relativ zum Mittelfuß zueinander elastisch und/oder gelenkartig biegbar sind und wobei die Befestigungseinrichtung (3) zum Befestigen des Halteschenkels (2) im Bereich des Mittelfußes ausgebildet ist und der Korrekturschenkel (4) durch ein Korrekturelement (23) gebildet wird, welches lösbar mit der Basisstruktur (5) verbunden werden kann.

2. Orthopädische Vorrichtung nach Anspruch 1, **dadurch gekennzeichnet, dass** eine zweite Befestigungseinrichtung (9) vorhanden ist, welche die Großzehe (19) bei bestimmungsgemäßem Gebrauch bezüglich der Basisstruktur (5) festlegt.

3. Orthopädische Vorrichtung nach Anspruch 1, **dadurch gekennzeichnet, dass** der Randkonturverlauf einen Bereich, in dem sich eine bei Hallux-Valgus-Fehlstellungen typische Pseudo-Exostose bilden kann, freilässt.

4. Orthopädische Vorrichtung nach einem der vorhergehenden Ansprüche, **dadurch gekennzeichnet, dass** sich die Basisstruktur (5) bei bestimmungsgemäßem Gebrauch zumindest unterhalb der Großzehe erstreckt.

5. Orthopädische Vorrichtung nach einem der vorhergehenden Ansprüche, **dadurch gekennzeichnet, dass** die Basisstruktur (5) im Wesentlichen flachplattenförmig ausgebildet ist.

6. Orthopädische Vorrichtung nach Anspruch 1, **dadurch gekennzeichnet, dass** die Biegezone (6) bei bestimmungsgemäßem Gebrauch in einem Bereich unterhalb zumindest eines Zehengrundgelenks angeordnet ist.

7. Orthopädische Vorrichtung nach Anspruch 1, **dadurch gekennzeichnet, dass** die Biegezone (6) durch ein Filmscharnier gebildet wird.

8. Orthopädische Vorrichtung nach einem der vorhergehenden Ansprüche, **dadurch gekennzeichnet, dass** die Vorrichtung zumindest aus einer ersten Komponente, z.B. einem ersten Kunststoffwerkstoff, und aus einer zweiten Komponente, z. B. einem zweiten Kunststoffwerkstoff, besteht, wobei die erste Komponente härter als die zweite Komponente ist.

9. Orthopädische Vorrichtung nach einem der Ansprüche 7 oder 8, **dadurch gekennzeichnet, dass** zumindest der Halteschenkel (2) und der Korrekturschenkel (4) aus der ersten Komponente und zumindest die Biegezone (6) aus der zweiten Komponente gebildet ist.

10. Orthopädische Vorrichtung nach einem der vorhergehenden Ansprüche, **dadurch gekennzeichnet, dass** der Korrekturschenkel (4) mittels einer zweiten Befestigungseinrichtung (9) an der zu korrigierenden Zehe befestigbar ist.

11. Orthopädische Vorrichtung nach einem der vorhergehenden Ansprüche, **dadurch gekennzeichnet, dass** der von dem Randkonturverlauf freigelassene Seitenbereich (16) einen Öffnungswinkel (α) zwischen 50° und 160°, vorzugsweise zwischen 70° und 130°, insbesondere von etwa 90° aufweist.

12. Orthopädische Vorrichtung nach einem der vorhergehenden Ansprüche, **dadurch gekennzeichnet, dass** der Korrekturschenkel (4) zwischen der Großzehe und der daneben liegenden zweiten Zehe angeordnet ist und als von der Basisstruktur (5) nach oben abgehender Steg ausgebildet ist.

## Claims

1. An orthopedic device, particularly for correcting toe misalignments, with a holding leg (2), a first fastening device (3), at least one correcting leg (4) for directly or indirectly acting on the big toe and a plate-like base structure (5) connecting the holding leg (2) to the correcting leg (4), the device having an edge curvature that with proper use, leaves open the inner foot side region (16) of the metatarsophalangeal joint of the big toe, **characterized in that**
Alternative 1:
the holding leg (2), the correcting leg (4), and the base structure (5) are made of a single piece and with proper use, the base structure (5) extends at least beneath the metatarsophalangeal joint of the big toe; the base structure (5) has or forms a bending zone (6); with proper use, the holding leg (2) and the correcting leg (4) are able to bend elastically and/or in articulated fashion in a natural bending direction of the toes relative to the metatarsus; and the fastening device (3) for fastening the holding leg (2) is located in the region of the metatarsus or
Alternative 2:
with proper use, the base structure (5) extends at least beneath the metatarsophalangeal joint of the big toe; the base structure (5) has or forms a bending zone (6); with proper use, the holding leg (2) and the correcting leg (4) are able to bend elastically and/or in articulated fashion in a natural bending direction of the toes relative to the metatarsus; the fastening device (3) for fastening the holding leg (2) is located in the region of the metatarsus; and the correcting leg (4) is composed of a correcting element (23) that can be detachably connected to the base structure (5).

2. The orthopedic device according to claim 1, **characterized in that** a second fastening device (9) is provided, which with proper use, immobilizes the big toe (19) relative to the base structure (5).

3. The orthopedic device according to claim 1, **characterized in that** the edge contour curve leaves open a region in which a pseudo-exotosis can form that is typical in hallux valgus misalignments.

4. The orthopedic device according to one of the preceding claims, **characterized in that** with proper use, the base structure (5) extends at least beneath the big toe.

5. The orthopedic device according to one of the preceding claims, **characterized in that** the base structure (5) is essentially embodied in the form of a flat plate.

6. The orthopedic device according to claim 1, **characterized in that** with proper use, the bending zone (6) is positioned in a region beneath at least one metatarsophalangeal joint.

7. The orthopedic device according to claim 1, **characterized in that** the bending zone (6) is composed of a film hinge.

8. The orthopedic device according to one of the preceding claims, **characterized in that** the device is composed of a first component such as a first synthetic material and of a second component such as a second synthetic material and the first component is harder than the second component.

9. The orthopedic device according to one of claims 7 or 8, **characterized in that** at least the holding leg (2) and the correcting leg (4) are composed of the first component and at least the bending zone (6) is composed of the second component.

10. The orthopedic device according to one of the preceding claims, **characterized in that** the correcting leg (4) can be fastened by means of a second fastening device (9) to the toe that is to be corrected.

11. The orthopedic device according to one of the preceding claims, **characterized in that** the side region (16) that is left open by the edge contour curve has an opening angle (α) of between 50° and 160°, preferably between 70° and 130°, and particular of approximately 90°.

12. The orthopedic device according to one of the preceding claims, **characterized in that** the correcting leg (4) is positioned between the big toe and the adjacent second toe and is embodied as a partition protruding upward from the based structure (5).

## Revendications

1. Dispositif orthopédique, destiné en particulier à la correction de déformations des orteils, comportant une branche de retenue (2), un premier moyen de fixation (3), au moins une branche de correction (4) pour agir directement ou indirectement sur le gros orteil et une structure de base (5) en forme de plaque reliant la branche de retenue (2) et la branche de correction (4), le dispositif présentant un contour de bord qui, lors d'une utilisation adéquate, laisse à découvert une zone latérale (16) côté intérieur du pied de l'articulation basale du gros orteil, **caractérisé en ce que**
variante 1 :
la branche de retenue (2), la branche de correction (4) et la structure de base (5) sont réalisées d'un seul tenant, et lors d'une utilisation adéquate, la structure de base (5) s'étend au moins au-dessous de l'articulation basale du gros orteil, la structure de base (5) possédant ou formant une zone de flexion (6), et lors d'une utilisation adéquate la branche de retenue (2) et la branche de correction (4) sont flexibles élastiquement et/ou en articulation l'une par rapport à l'autre dans une direction de flexion naturelle des orteils par rapport au métatarse, et le moyen de fixation (3) destiné à fixer la branche de retenue (2) est réalisé dans la zone du métatarse, ou
variante 2 :
lors d'une utilisation adéquate, la structure de base (5) s'étend au moins au-dessous de l'articulation basale du gros orteil, la structure de base (5) possédant ou formant une zone de flexion (6), et lors d'une utilisation adéquate la branche de retenue (2) et la branche de correction (4) sont flexibles élastiquement et/ou en articulation l'une par rapport à l'autre dans une direction de flexion naturelle des orteils par rapport au métatarse, et le moyen de fixation (3) destiné à fixer la branche de retenue (2) est réalisé dans la zone du métatarse et la branche de correction (4) est formée par un élément de correction (23) qui peut être relié de façon détachable à la structure de base (5).

2. Dispositif orthopédique selon la revendication 1, **caractérisé en ce qu'**il existe un second moyen de fixation (9) qui, lors d'une utilisation adéquate, immobilise le gros orteil (19) par rapport à la structure de base (5).

3. Dispositif orthopédique selon la revendication 1, **caractérisé en ce que** le contour de bord laisse à découvert une zone dans laquelle peut se former une pseudo-exostose typique pour des déformations d'hallux-valgus.

4. Dispositif orthopédique selon l'une des revendications précédentes, **caractérisé en ce que** la structure de base (5) s'étend, lors d'une utilisation adéquate, au moins au-dessous du gros orteil.

5. Dispositif orthopédique selon l'une des revendications précédentes, **caractérisé en ce que** la structure de base (5) est réalisée sensiblement en forme de plaque plane.

6. Dispositif orthopédique selon la revendication 1, **caractérisé en ce que** la zone de flexion (6) est agencée, lors d'une utilisation adéquate, dans une zone au-dessous d'au moins une articulation basale d'orteil.

7. Dispositif orthopédique selon la revendication 1, **caractérisé en ce que** la zone de flexion (6) est formée par une charnière en film.

8. Dispositif orthopédique selon l'une des revendications précédentes, **caractérisé en ce que** le dispositif est constitué d'au moins une première composante, par exemple d'une première matière plastique, et d'une seconde composante, par exemple d'une seconde matière plastique, la première composante étant plus dure que la seconde composante.

9. Dispositif orthopédique selon l'une des revendications 7 ou 8, **caractérisé en ce qu'**au moins la branche de retenue (2) et la branche de correction (4) sont formées de la première composante, et au moins la zone de flexion (6) est formée de la seconde composante.

10. Dispositif orthopédique selon l'une des revendications précédentes, **caractérisé en ce que** la branche de correction (4) peut être fixée sur l'orteil à corriger à l'aide d'un second moyen de fixation (9).

11. Dispositif orthopédique selon l'une des revendications précédentes, **caractérisé en ce que** la zone latérale (16) laissée à découvert par le contour de bord présente un angle d'ouverture (α) compris entre 50° et 160°, de préférence entre 70° et 130°, en particulier d'environ 90°.

12. Dispositif orthopédique selon l'une des revendications précédentes, **caractérisé en ce que** la branche de correction (4) est agencée entre le gros orteil et le second orteil situé à côté et est réalisée en tant que barrette s'étendant à partir de la structure de base (5) vers le haut.
